# FASCICULE DE BREVET EUROPEEN

(11) **EP 1 708 994 B1**
(45) Date de publication et mention de la délivrance du brevet: **08.08.2007**
(21) Numéro de dépôt: 05717376.7
(22) Date de dépôt: 07.01.2005
(51) Int. Cl.: C07D 207/08, C07D 211/46, C07D 211/22, C07D 213/65, C07D 215/28, C07D 215/20, C07D 217/24, C07C 271/16, C07D 401/12, A61K 31/27, A61K 31/40, A61K 31/445, A61K 31/4709, A61P 1/00, A61P 1/08, A61P 3/04, A61P 9/00, A61P 11/00, A61P 13/00, A61P 25/00, A61P 35/00

(54) **DERIVES DE TYPE ARYLOXYALKYLCARBAMATES, LEUR PREPARATION ET LEUR APPLICATION EN THERAPEUTIQUE**
DERIVATE VOM ARYLOXYALKYLCARBAMAT-TYP, VERFAHREN ZU DEREN HERSTELLUNG UND DEREN VERWENDUNG IN THERAPEUTIKA
ARYLOXYALKYLCARBAMATE-TYPE DERIVATIVES, PREPARATION METHOD THEREOF AND USE OF SAME IN THERAPEUTICS

(30) Priorité: 16.01.2004 FR 0400389
(43) Date de publication de la demande: 11.10.2006
(73) Titulaire: Sanofi-Aventis, 75013 Paris (FR)
(72) Inventeur: ABOUABDELLAH, Ahmed, F-94320 Thiais (FR); ALMARIO GARCIA, Antonio, F-92290 Chatenay Malabry (FR); FROISSANT, Jacques, 41160 Brevainville (FR); HOORNAERT, Christian, F-92160 Antony (FR)
(74) Mandataire: Morel-Pécheux, Muriel
(86) Numéro de dépôt international: PCT/FR2005/000028
(87) Numéro de publication internationale: WO 2005/077898

(56) Documents cités:
- WO-A-02/087569
- WO-A-03/065989
- WO-A-20/04067498
- TARZIA G ET AL: "Design, synthesis, and structure-activity relationships of alkylcarbamic acid aryl esters, a new class of Fatty Acid Amide Hydrolase inhibitors" JOURNAL OF MEDICINAL AND PHARMACEUTICAL CHEMISTRY, AMERICAN CHEMICAL SOCIETY. EASTON, US, vol. 46, no. 12, 2003, pages 2352-2360, XP002257137

## Description

L'invention a pour objet des dérivés d'aryloxyalkylcarbamates, leur préparation et leur supplication en thérapeutique.

Les composés de l'invention répondent à la formule générale (I) : 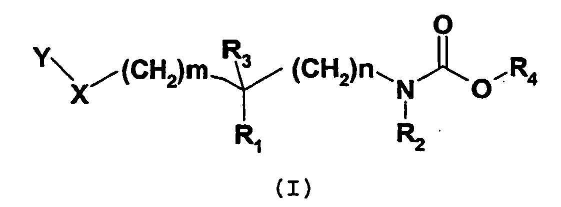 dans laquelle
m représente 0, 1, 2 ou 3 ;
n représente 0, 1, 2 ou 3 ;
X représente un atome d'oxygène ou de soufre ou un groupe SO ou SO₂ ;
R₁ et R₂ représentent, indépendamment l'un de l'autre, un atome d'hydrogène ou un groupe C₁₋₃-alkyle, ou R₁ et R₂ forment ensemble un groupe -(CH₂)ₚ-, où p représente un nombre entier allant de 1 à 5 tel que n + p soit un nombre entier allant de 2 à 5 ;
R₃ représente un atome d'hydrogène ou de fluor ou un groupe hydroxy ou méthyle ;
R₄ représente un groupe de formule générale CHR₅CONHR₆ dans laquelle
R₅ représente un atome d'hydrogène ou un groupe C₁₋₆-alkyle et
R₆ représente un atome d'hydrogène ou un groupe C₁₋₆-alkyle, C₃₋₇-cycloalkyle, C₃₋₇-cycloalkyle-C₁₋₆-alkylène ;
Y représente
   un groupe Y₁ choisi parmi notamment un phényle, pyridinyle, pyridazinyle, pyrimidinyle, pyrazinyle, triazinyle, thiazolyle, naphtyle, quinolinyle, isoquinolinyle, phtalazinyle, quinazolinyle, quinoxalinyle, naphtyridinyle, cinnolinyle, benzofuranyle, dihydrobenzofuranyle, benzo-thiényle, dihydrobenzot hiényle, indolyle, isoindolyle, indolinyle, benzimidazolyle, benzoxazolyle, benzisoxazolyle, benzothiazolyle, benzisothiazolyle, benzotriazolyle, benzoxadiazolyle, benzothiadiazolyle ; le groupe Y₁ étant éventuellement substitué par un ou plusieurs substituants Y₂ identiques ou différents l'un de l'autre ou par un groupe Y₃; Y₂ représente un atome d'halogène, un groupe cyano, nitro, C₁₋₈-alkyle, C₁₋₈-alcoxy, C₁₋₈-thioalkyle, C₁₋₈-fluoroalkyle, C₁₋₈-fluoroalcoxy, C₁₋₈-fluorothioalkyle, C₃₋₇-cycloalkyle, C₃₋₇-cycloalkyloxy, C₃₋₇-cycloalkyle-C₁₋₈-alkylène,
   C₃₋₇-cycloalkyle-C₁₋₈-alkyloxy, hydroxy, NR₇R₈, NHCOR₇, NHSO₂R₇, COR₇, CO₂R₇, CONR₇R₈, SO₂R₇, SO₂NR₇R₈, -O- (C₁₋₃-alkylène)-O-, phényloxy, phénylthio, phényl-C₁-C₈-alkylène, phényl-C₁-C₈-alkyloxy ou phényl-C₁-C₈-alkylthio ;
Y₃ représente un groupe choisi parmi notamment un phényle, pyridinyle, pyrimidinyle, pyrazinyle ou pyridazinyle ; le ou les groupes Y₃ pouvant être substitués par un ou plusieurs groupes Y₂ identiques ou différents l'un de l'autre ;
R₇ et R₈ représentent indépendamment l'un de l'autre un atome d'hydrogène ou un groupe C₁₋₆-alkyle, ou forment avec l'atome d'azote qui les porte un cycle azétidine, pyrrolidine, pipéridine, morpholine, thiomorpholine, azépine, pipérazine éventuellement substitué par un groupe C₁₋₃-alkyle ou benzyle.

Parmi les composés de formule générale (I), un premier groupe de composés est celui pour lequel :
Y représente
un groupe Y₁ choisi parmi notamment un phényle, pyridinyle, pyrimidinyle, thiazolyle, naphtyle, quinolinyle, isoquinolinyle, benzoxazolyle ; le groupe Y₁ étant éventuellement substitué par un ou plusieurs substituants, plus particulièrement par un ou deux substituants, Y₂ identiques ou différents l'un de l'autre ou par un groupe Y₃; Y₂ représente un atome d'halogène, plus particulièrement un chlore, un fluor ou un brome, un groupe cyano, C₁₋₈-alkyle, plus particulièrement un méthyle, isopropyle, butyle, ter-butyle ou tétraméthylbutyle, C₁₋₈-alcoxy, plus particulièrement un méthoxy, éthoxy ou propoxy, C₁₋₈-fluoroalkyle, plus particulièrement un trifluorométhyle, C₁₋₈-fluoroalcoxy, plus particulièrement un trifluorométhoxy, phényloxy, phényl-C₁-C₈-alkylène, plus particulièrement un phényle-(1,1-diméthylméthylène) ;
Y₃ représente un groupe phényle ; Y₃ pouvant être substitué par un ou plusieurs groupes, plus particulièrement par un ou deux groupes, Y₂ identiques ou différents l'un de l'autre.

Parmi les composés du premier groupe tel que défini ci-dessus, un second groupe de composés est celui pour lequel :
Y représente
un groupe Y₁ choisi parmi notamment un phényle ou un naphtyle ; le groupe Y₁ étant éventuellement substitué par un ou plusieurs substituants, plus particulièrement par un ou deux substituants, Y₂ identiques ou différents l'un de l'autre ou par un groupe Y₃ ;
Y₂ représente un atome d'halogène, plus particulièrement un chlore, un fluor ou un brome, un groupe cyano, C₁₋₈-alkyle, plus particulièrement un méthyle, isopropyle, butyle, terbutyle ou tétraméthylbutyle, C₁₋₈-alcoxy, plus particulièrement un méthoxy, éthoxy ou propoxy, C₁₋₈-fluoroalkyle, plus particulièrement un trifluorométhyle, C₁₋₈-fluoroalcoxy, plus particulièrement un trifluorométhoxy, phényloxy, phényl-C₁-C₈-alkylène, plus particulièrement un phényle-(1,1-diméthylméthylène) ;
Y₃ représente un groupe phényle ; Y₃ pouvant être substitué par un ou plusieurs groupes, plus particulièrement par un ou deux groupes, Y₂ identiques ou différents l'un de l'autre.

Parmi les composés de formule générale (I), un troisième groupe de composés est celui pour lequel:
m représente 0, 1, 2 ou 3 ; et/ou
n représente 0, 1, 2 ou 3 ; et/ou
R₁ et R₂ représentent, indépendamment l'un de l'autre, un atome d'hydrogène ou un groupe C₁₋₃-alkyle, ou R₁ et R₂ forment ensemble un groupe -(CH₂)ₚ-, où p représente un nombre entier allant de 1 à 5 tel que n + p soit un nombre entier allant de 2 à 5 ;
avec la condition que quand R₁ et R₂ représentent, indépendamment l'un de l'autre, un atome d'hydrogène ou un groupe C₁₋₃-alkyle, alors m + n > 1.

Parmi les composés du troisième groupe tel que défini ci-dessus, un quatrième groupe de composés est celui pour lequel :
m représente 0, 1, 2 ou 3 ; et/ou
n représente 0, 1, 2 ou 3 ; et/ou
R₁ et R₂ forment ensemble un groupe -(CH₂)ₚ-, où p représente un nombre entier allant de 1 à 4 tel que n + p soit égal à 4.

Parmi les composés de formule générale (T), un cinquième groupe de composés est celui pour lequel X représente un atome d'oxygène.

Parmi les composés de formule générale (I), un sixième groupe de composés est celui pour lequel R₃ représente un atome d'hydrogène.

Un septième groupe est formé des composés pour lesquels à la fois R₁, R₂, R₃, R₄, R₅, R₆, R₇, R₈, X, Y, Y₁, Y₂, Y₃, n et m sont tels que définis dans les sous-groupes de composés ci-dessus.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention. Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₃-alkyle représente une chaîne carbonée de 1 à 3 atomes de carbone, linéaire ou ramifiée, plus particulièrement un méthyle, éthyle, propyle, 1-méthyléthyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène, 1,1-diméthylméthylène ;
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle ;
- alcènylène, un groupe aliphatique à 2 carbones, insaturé divalent, plus particulièrement un éthylène ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor ;
- atome d'halogène, un fluor, un chlore, un brome ou un iode.

Les composés de l'invention peuvent être préparés selon différentes méthodes, illustrées par les schémas qui suivent.

Ainsi une méthode de préparation (schéma 1) consiste à faire réagir une amine de formule générale (II), dans laquelle Y, X, R₁, R₂, R₃, m et n sont tels que définis dans la formule générale (I), avec un carbonate de formule générale (III), dans laquelle Z représente un atome d'hydrogène ou un groupement nitro, R₅ est tel que défini dans la formule générale (I) et R représente un groupe méthyle ou éthyle, dans un solvant tel que le toluène ou le dichloroéthane, à une température comprise entre 0 et 80°C. Les carbamate-esters de formule générale (IV) ainsi obtenus sont ensuite transformés en composés de formule générale (I) par aminolyse au moyen d'une amine de formule générale R₆NH₂ où R₆ est tel que défini dans la formule générale (I). La réaction d'aminolyse peut être réalisée dans un solvant tel que le méthanol ou l'éthanol, ou un mélange de solvants tels que le méthanol et le tétrahydrofurane.

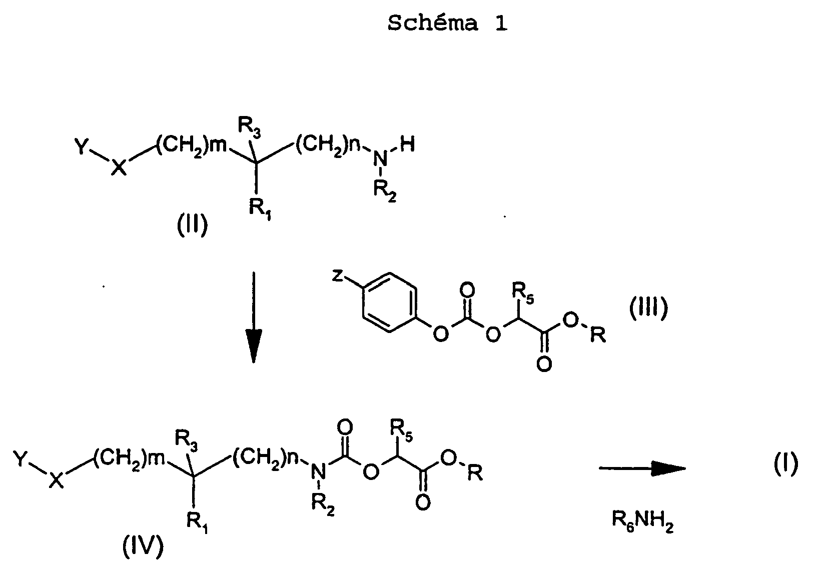

Une autre méthode (schéma 2) pour obtenir les composés de formule générale (I) dans laquelle R₂ représente plus particulièrement un atome d'hydrogène, consiste à faire réagir un dérivé de formule générale (IIa) dans laquelle W représente un groupement hydroxyle, mésylate, tosylate, ou un atome de chlore, de brome ou d'iode, et dans laquelle Y, X, R₁, R₃, m, et n sont tels que définis dans la formule générale (I), avec une oxazolidine-dione de structure générale (V) dans laquelle R₅ est tel que défini dans la formule générale (I), pour fournir le dérivé oxazolidine-dione de structure générale (VI). Dans le cas où W représente un groupement hydroxyle, la réaction peut être conduite suivant les conditions de Mitsunobu (Synthesis, 1981, 1-28), par exemple, par action d'azodicarboxylate de diéthyle ou de diisopropyle en présence de triphénylphosphine. Dans le cas où W représente un atome de chlore, de brome, ou d'iode, ou un groupement mésylate ou tosylate, la réaction peut être conduite en présence d'une base telle que la 1,1,3,3-tétraméthylguanidine, l'hydrure de sodium ou le *tert*-butylate de sodium dans un solvant tel que le tétrahydrofurane, l'acétonitrile ou le diméthylformamide à une température comprise entre 0°C et la température de reflux du solvant. Le dérivé oxazolidine-dione de formule générale (VI) ainsi obtenu est ensuite transformé en composé de formule générale (I), par aminolyse au moyen d'une amine de formule générale R₆NH₂ où R₆ est tel que défini dans la formule générale (I).

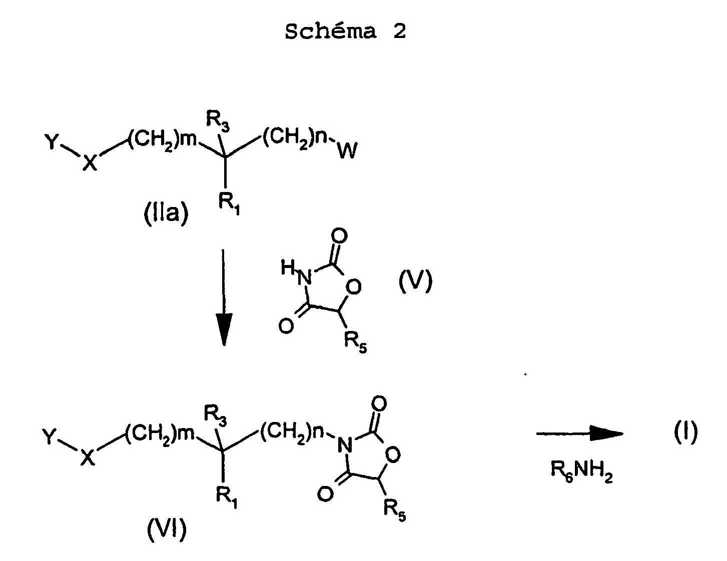

Une autre variante (schéma 3) pour obtenir les composés de formule générale (I) dans laquelle X représente plus particulièrement un atome d'oxygène, consiste à faire réagir un dérivé alcool de formule générale (VIIa), (VIIb) ou (VIIc) avec un dérivé de phénol de structure générale YOH dans laquelle le Y est tel que défini dans la formule générale (I), par exemple suivant les conditions de réaction de Mitsunobu (Synthesis, 1981, 1-28) ou modifiées (Tetrahedron Letters 1993, 34, 1639-1642), les dérivés carbamates-ester (IVa) et oxazolidinedione (VIa) étant ensuite transformés en composés de formule générale (I) par réaction d'aminolyse au moyen d'une amine de structure générale R₆NH₂ où R₆ est tel que défini dans la formule générale (I).

Dans les formules générales (VIIa), (VIIb) et (VIIc), les groupes R₁, R₂, R₃, R₅, R₆, m, n, et R sont tels que définis ci-dessus.

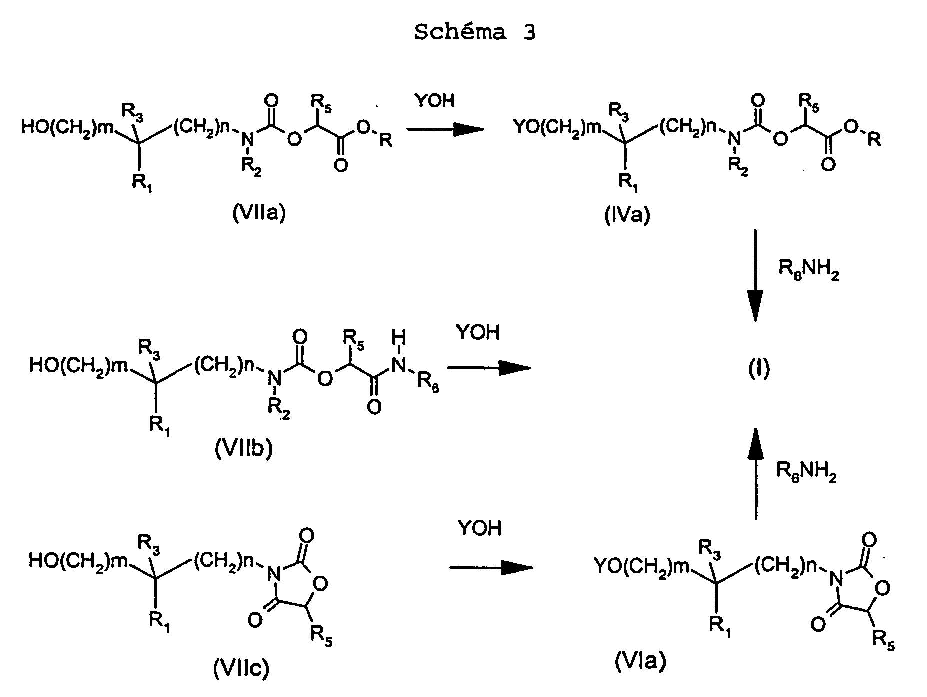

Une autre variante (schéma 4) pour obtenir les composés de formule générale (I) dans laquelle Y représente plus particulièrement un groupement Y₁-Y₃ de type aryle-aryle, aryle-hétéroaryle, hétéroaryle-aryle ou hétéroaryle-hétéroaryle, consiste à faire réagir un dérivé d'halogénure d'aryle de structure générale (VIII), dans laquelle U est un atome de brome ou d'iode et Y₁, X₁, R₁, R₂, R₃, R₅, R₆, n et m sont tels que définis dans la formule générale (I), avec un dérivé d'acide aryl- ou hétéroaryl-boronique de formule Y₃B(OH)₂, où Y₃ est tel que défini dans la formule générale (I), suivant les conditions de réaction de Suzuki (Chem. Rev. 1995, 95, 2457-2483) ou avec un dérivé d'aryl- ou d'hétéroaryl-tri-alkylstannane de formule Y₃Sn(R')₃, où Y₃ est tel que défini dans la formule générale (I) et R' est un C₁₋₄-alkyle, suivant les conditions de réaction de Stille (Angew. Chem. Int. Ed. 1986, 25, 504-524).

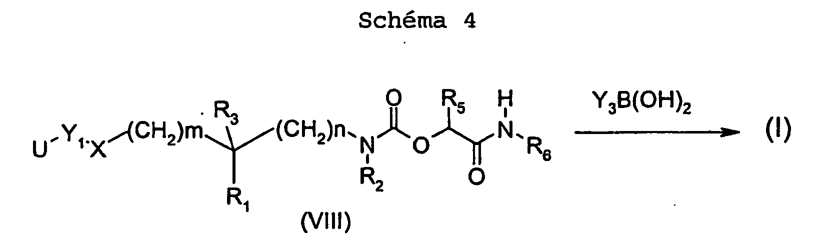

Les composés de formules générales (II), (IIa), (III), (V), (VIIa), (VIIb), (VIIc), (VIII) et les dérivés de phénol de structure générale YOH, quand leur mode de préparation n'est pas décrit, sont disponibles dans le commerce ou décrits dans la littérature, ou bien peuvent être préparés selon des méthodes qui y sont décrites ou qui sont connues de l'homme du métier.
Les amines de formule générale R₆NH2 sont disponibles dans le commerce.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.

Les numéros indiqués entre parenthèses dans les titres des exemples correspondent à ceux de la 1ère colonne du tableau ci-après.
La nomenclature UICPA (Union Internationale de Chimie Pure et Appliquée - IUPAC en anglais) a été utilisée pour la dénomination des composés dans les exemples suivants. Par exemple, pour le groupe biphényle, la numérotation suivante a été respectée:

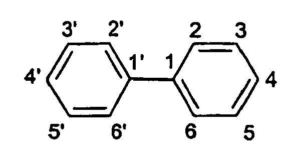

### Exemple 1 (composé n°1)

### {2-[(4-chlorophényl)oxy]éthyl}carbamate de 2-(méthylamino)-2-oxoéthyle

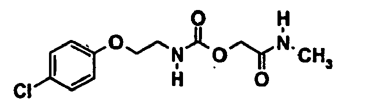

### 1.1. [(phényloxycarbonyl)oxy]acétate d'éthyle

A une solution de 25 g (240 mmoles) de glycolate d'éthyle et de 55 ml (315 mmoles) de diisopropyléthylamine dans 500 ml de toluène, on ajoute lentement à température ambiante 32 ml (256 mmoles) de chloroformiate de phényle. On poursuit l'agitation à température ambiante pendant 2 heures. On sépare le sel formé et on concentre le filtrat sous pression réduite. On obtient 53,7 g de produit huileux utilisé tel quel, dans l'étape suivante.

### 1.2. {[({2-[(4-chlorophényl)oxy]éthyl}amino)carbonyl] oxy}acétate d'éthyle

On chauffe à 60°C pendant une nuit une solution de 0,6 g (3,5 mmoles) de [(4-chlorophényl)oxy]éthylamine (Chim. Ther. 1973, 8, 259-270) et de 1,3 g (5,8 mmoles) de [(phényloxycarbonyl)oxy]acétate d'éthyle, préparé à l'étape 1.1., dans 30 ml de toluène. On évapore à sec et on purifie le produit par chromatographie sur gel de silice en éluant par un mélange 30/70 d'acétate d'éthyle et de cyclohexane. On obtient 0,7 g de produit huileux contenant ~10% de produit cyclisé oxazolidine-dione, utilisé tel quel dans l'étape suivante.

### 1.3. {2-[(4-chlorophényl)oxy]éthyl}carbamate de 2-(méthylamino)-2-oxoéthyle

On ajoute 3,5 ml (7 mmoles) d'une solution de méthylamine 2M dans le tétrahydrofurane à une solution de 0,7 g (2,3 mmoles) de {[({2-[(4-chlorophényl)oxy]éthyl}amino) carbonyl]oxy}acétate d'éthyle, préparé à l'étape 1.2., dans 5 ml de méthanol. On laisse réagir une nuit à température ambiante. On évapore à sec et on lave le solide résiduel par de l'hexane puis du diisopropyléther pour obtenir 0,59 g de produit sous forme de poudre.
Point de fusion (°C) : 147-149
LC-MS : M+H = 287
RMN-¹H (DMSO) δ (ppm) : 7,75 (m, 1H), 7,40 (m, 1H), 7,25 (d, 2H), 6,95 (d,2H), 4,35 (s,2H), 3,95 (t,2H), 3,35 (m,2H), 2,60 (d,3H)

### Exemple 2 (composé n° 11)

### (2-[(4-cyanophényl)oxy]éthyl)carbamate de 2-amino-2-oxoéthyle

### 2.1. 3-(2-hydroxyéthyl)-1,3-oxazolidine-2,4-dione

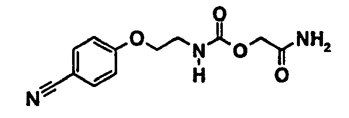

On ajoute goutte à goutte, en 2 heures, une solution de 3 ml (39,6 mmoles) de glycolate de méthyle dans 25 ml de tétrahydrofurane, à une solution de 49 ml (95 mmoles) de phosgène 1, 9M dans le toluène diluée dans 50 ml de tétrahydrofurane et refroidie par un bain de glace. On agite ensuite à température ambiante pendant 16 heures et on évapore à sec. On coévapore 4 fois avec 30 ml de dichlorométhane. On reprend le résidu dans 40 ml d'acétonitrile et on l'ajoute goutte à goutte, en 1 heure, à une solution de 3,4 ml (59,4 mmoles) d'éthanolamine et de 30 ml (178 mmoles) de diisoproyléthylamine dans un mélange 50/10 d'acétonitrile et de dichlorométhane refroidie par un bain de glace. On agite ensuite à température ambiante pendant 16 heures. On filtre sur célite, on évapore à sec et on purifie le produit par chromatographie sur gel de silice en éluant par un mélange 70/30 puis 80/20 d'acétate d'éthyle et de n-hexane pour obtenir 4,9 g de produit sous forme de solide blanc.

### 2.2. (2-[(4-cyanophényl)oxy]éthyl)carbamate de 2-amino-2-oxoéthyle

On ajoute goutte à goutte 0,61 ml (1,35 mmoles) d'une solution 2,2M de azodicarboxylate de diéthyle dans le toluène à une solution de 0,13 g (0,88 mmoles) de 3-(2-hydroxyéthyl)-1,3-oxazolidine-2,4-dione, préparé à l'étape 2.1., de 0,35 g (1,35 mmoles) de triphénylphosphine et de 0,10 g (0,89 mmoles) de 4-hydroxybenzonitrile dans 2 ml de benzène refroidie par un bain de glace. On agite ensuite le mélange réactionnel à température ambiante pendant 16 heures. On évapore à sec et on purifie le produit par chromatographie sur gel de silice en éluant par un mélange 99/1 puis 98/2 de dichlorométhane et d'acétate d'éthyle. On reprend le produit dans 1,5 ml d'une solution d'ammoniac 7M (10,5 mmoles) dans le méthanol. On agite pendant une heure. On filtre le précipité et on le lave avec de l'acétate d'éthyle pour obtenir 0,035 g de solide blanc.
Point de fusion (°C) : 204-206
LC-MS : M+H = 264
RMN-¹H (DMSO) δ (ppm): 7, 55 (d, 2H), 7,05 (m, 1H), 6, 90-6, 80 (m+d, 4H), 4,35 (s,2H), 4,05 (t,2H), 3,45 (m,2H)

### Exemple 3 (composé n° 58)

### [4-(1-naphtalènyloxy]butyl]carbamate de 2-amino-2-oxoéthyle

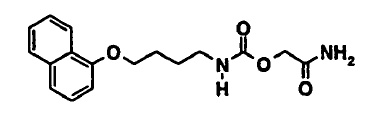

3.1. 3-[4-(1-naphtalènyloxy]butyl]-1,3-oxazolidine-2,4-dione A une solution de 3,1 g (11,1 mmoles) de 1-[(4-bromobutyl)oxy]naphtalène (Eur. J. Med. Chem. 1997, 32, 175-179) et de 1,35 g (13,3 mmoles) de 1,3-oxazolidine-2,4-dione (J. Med. Chem. 1991, 34, 1542-1543) dans 30 ml de tétrahydrofurane, on ajoute goutte à goutte une solution de 2,55 g (22,2 mmoles) de 1,1,3,3-tétraméthylguanidine dans 15 ml de tetrahydrofurane. On chauffe au reflux pendant 8 heures. On rajoute 0,28 g (2,7 mmoles) de 1,3-oxazolidin-2,4-dione et 0,32 g (2,7 mmoles) de 1,1,3,3-tétraméthylguanidine et on chauffe au reflux pendant 4 heures supplémentaires. On refroidit le mélange réactionnel par un bain de glace et on ajoute 100 ml d'acétate d'éthyle puis 50 ml d'acide chlorhydrique aqueux 1M. On décante, on extrait la phase aqueuse par 2 x 80 ml d'acétate d'éthyle. On lave ensuite les phases organiques par 80 ml d'eau puis 80 ml d'une solution aqueuse saturée en chlorure de sodium. On les sèche sur sulfate de sodium puis on évapore à sec. On purifie le produit par chromatographie sur gel de silice en éluant par un mélange 80/20 de cyclohexane et d'acétate d'éthyle pour obtenir 2,0 g de produit utilisé tel quel dans l'étape suivante.

### 3.2. [4-(1-naphtalènyloxy]butyl]carbamate de 2-amino-2-oxoéthyle

On dissout 1,50 g (5,0 mmoles) de 3-[4-(1-naphtalènyloxy]butyl]-1,3-oxazolidine-2,4-dione, préparé à l'étape 3.1., dans un mélange de 10 ml de tetrahydrofurane et de 28 ml d'une solution d'ammoniac 7 N (200 mmoles) dans le méthanol. On laisse réagir une nuit à température ambiante puis on évapore à sec. On purifie le produit par chromatographie sur gel de silice en éluant par un mélange 97/3 de dichlorométhane et de méthanol. On recristallise dans l'acétate d'éthyle puis on lave par du diéthyléther pour obtenir 0,73 g de produit sous forme de solide blanc.
Point de fusion (°C) : 80-82
LC-MS : M+H = 317
RMN-¹H (CDCl₃) δ (ppm) : 8,25 (dd, 1H), 7,80 (dd, 1H), 7, 55-7, 30 (m, 4H), 6, 80 (d, 1H), 6,00 (m, 1H), 5,65 (m, 1H), 5,05 (m, 1H), 4,65 (s,2H), 4,20 (t,2H), 3,35 (m,2H), 2,00 (m,2H), 1,90 (m,2H)

### Exemple 4 (composé n° 85)

### 4-[(4'-fluoro-4-biphényl)oxy]-1-pipéridinecarboxylate de 2-(méthylamino)-2-oxoéthyle

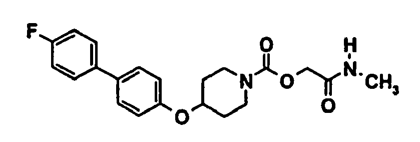

### 4.1. 4-[(4-bromophényl)oxy]-1-pipéridinecarboxylate de 1,1-diméthyléthyle

A une solution de 2,01 g (10 mmoles) de 4-hydroxy-1-pipéridinecarboxylate de 1,1-diméthyléthyle dans 20 ml de diméthylformamide, on ajoute 7 g (40 mmoles) de 1-bromo-4-fluorobenzène et 2,5 g (50 mmoles) d'hydrure de sodium à 50% dans l'huile minérale. On agite le mélange à 100°C pendant 3 heures, puis on évapore à sec. On reprend le résidu par 50 ml d'eau glacée et on extrait avec du dichlorométhane. On évapore à sec les extraits organiques pour obtenir 3,5 g d'un produit huileux utilisé tel quel dans l'étape suivante.

### 4.2. 4-[(4-bromophényl)oxy]pipéridine

A une solution de 3,5 g (9,83 mmoles) de 4-[(4-bromophényl)oxy]-1-pipéridinecarboxylate de 1,1-diméthyléthyle, préparé à l'étape 4.1., dans 20 ml de dichlorométhane, on ajoute 10 ml d'acide trifluoroacétique et on agite la solution à température ambiante pendant 1 heure. On évapore à sec puis on reprend le résidu par 30 ml de toluène que l'on évapore à nouveau à sec. On lave ensuite le résidu avec du pentane puis on le reprend dans un mélange de 60 ml de dichlorométhane et de 20 ml de solution aqueuse 4N d'ammoniaque. On agite vigoureusement pendant 15 minutes puis on décante la phase organique, on la sèche sur sulfate de sodium et on l'évapore à sec pour obtenir 2,7 g de produit sous forme d'huile utilisé tel quel dans l'étape suivante.

### 4.3 4-[(4-bromophényl)oxy]-1-pipéridinecarboxylate de 2-(éthyloxy)-2-oxoéthyle

On mélange 2,7 g (7,58 mmoles) de 4-[(4-bromo phényl)oxy]pipéridine, préparé à l'étape 4.2., et 1,70 g (7,6 mmoles) de {[(phényloxy)carbonyl]oxy}acétate d'éthyle, préparé suivant l'exemple 1.1, dans 40 ml de toluène et on chauffe la solution à 50°C pendant 20 heures. Après refroidissement, on évapore à sec et on purifie le produit par chromatographie sur gel de silice en éluant avec un mélange 40/60 d'acétate d'éthyle et de cyclohexane. On triture ensuite dans le diisopropyléther pour obtenir 2,9 g de produit sous forme de poudre.
Point de fusion (°C) : 87-88

### 4.4 4-[(4-bromophényl)oxy]-1-pipéxidinecarboxylate de 2-(méthylamino)-2-oxoéthyle

On agite à température ambiante pendant 20 heures 2,9 g (7,5 mmoles) de 4-[(4-bromophényl)oxy]-1-pipéridinecarboxylate de 2-(éthyloxy)-2-oxoéthyle, préparé à l'étape 4.3., dissous dans 10 ml d'une solution éthanolique à 33% de méthylamine. Après évaporation, on purifie le produit par chromatographie sur gel de silice en éluant avec de l'acétate d'éthyle pour obtenir 0,8 g de produit sous forme de gomme, utilisé tel quel dans l'étape suivante.

### 4.5. 4-[(4'-fluoro-4-biphényl)oxy]-1-pipéridinecarboxylate de 2-(méthylamino)-2-oxoéthyle

On place dans un tube en verre avec bouchon 0,1 g (0,27 mmoles) de 4-[(4-bromophényl)oxy]-1-pipéridinecarboxylate de 2- (méthylamino)-2-oxoéthyle, préparé à l'étape 4.4., 0,01 g de tetrakis (triphénylphosphine) palladium (0) et 0,057 g (0,4 mmoles) d'acide 4-fluorophénylboronique. On ajoute 4 ml de toluène, 2 ml d'une solution aqueuse 2N de carbonate de sodium et 0,5 ml d'éthanol. On chauffe à 80°C sous agitation pendant 2 heures. Après refroidissement, on ajoute 1 ml d'eau et 2 ml de toluène. On prélève la phase organique et on purifie le produit par chromatographie sur gel de silice en éluant avec un mélange 95/5 de dichlorométhane et de méthanol. On redissout le produit dans 1 ml d'éthanol puis on le reprécipite par ajout de 2 ml d'eau pour obtenir 0,031 g de produit sous forme de poudre.
Point de fusion (°C) : 117-119
LC-MS : M+H 387
RMN-¹H (CDCl₃) δ (ppm) : 7, 70 (dd, 2H) ; 7,65 (d, 2H) ; 7, 30 (dd, 2H) ; 7,20 (d, 2H); 6,25 (s large, 1H), 4, 80 (s + m, 3H) ; 4,00 - 3,70 (m, 4H); 3,05 (d, 3H); 2,25 - 2,00 (m, 4H)

### Exemple 5 (composé n° 120)

### 4-{[(4-bromophényl)oxy]méthyl}-1-pipiridinecarboxylate de 2-(méthylamino)-2-oxoéthyle

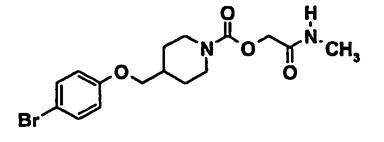

### 5.1. 4-{[(4-bromophényl)oxy]méthyl}-1-pipéridinecarboxylate de 1,1-diméthyléthyle

On procède comme décrit à l'exemple 4.1. A partir de 2,5 g (11,6 mmoles) de 4-(hydroxyméthyl)-1-pipéridinecarboxylate de 1,1-diméthyléthyle et de 8,13 g (46,4 mmoles) de 1-bromo-4-fluorobenzène, on obtient 5,75 g de produit brut sous forme d'huile.

### 5.2. 4-{[(4-bromophényl)oxy]méthyl}pipéridine

on procède comme décrit à l'exemple 4.2. A partir de 5,75 g de 4-{[(4-bromophényl)oxy]méthyl}-1-pipéri dinecarboxylate de 1,1-diméthyléthyle, préparé à l'étape 5.1, on obtient 3 g de produit sous forme d'huile.

### 5.3. 4-{[(4-bromophényl)oxy]méthyl}-1-pipéridinecarboxylate de 2-(éthyloxy)-2-oxoéthyle

On procède comme décrit à l'exemple 4.3. A partir de 1,6 g (5,9 mmoles) de 4-{[(4-bromophényl)oxy]méthyl}pipéridine, préparé à l'étape 5.2, et de 1,32 g (5,9 mmoles) de {[(phényloxy) carbonyl]oxy}acétate d'éthyle, préparé suivant l'exemple 1.1, on obtient le produit sous forme d'huile.

### 5.4. 4-{[(4-bromophényl)oxy]méthyl}-1-pipéridinecarboxylate de 2-(méthylamino)-2-oxoéthyle

On procède comme décrit à l'exemple 4.4. A partir de 4-{ [(4-bromophényl)oxy] méthyl}-1-pipéridinecarboxylate de 2-(éthyloxy)-2-oxoéthyl, préparé à l'étape 5.3, on obtient 1,1 g de produit sous forme de poudre.
Point de fusion (°C) : 163-165
LC-MS : M+H = 386
RMN-¹H (CDCl₃) δ (ppm) : 7,35 (d, 2H) ; 6,75 (d, 2H); 6,05 (s large, 1H); 4,70 - 4,50 (m, 2H); 4,30 - 4,10 (m, 2H); 3,80 (d, 2H); 3,00 - 2,75 (m, 2H); 2,85 (d, 3H); 2,10 - 1,80 (m, 3H); 1,45 - 1,20 (m, 2H)

### Exemple 6 (composé n° 154)

### 4-{ [(4'-(trifluorométhyl)-4-biphényl)oxy]méthyl}-1-pipéridinecarboxylate de 2-(méthylamino)-2-oxoéthyle

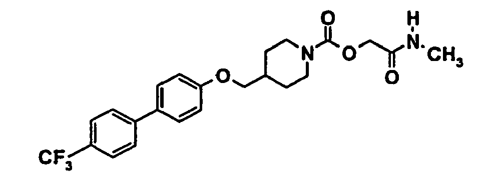

On procède comme décrit à l'exemple 4.5. A partir de 0,1 g (0,26 mmoles) de 4-{[(4-bromophényl)oxy]methyl}-1-pipéridinecarboxylate de 2-(méthylamino)-2-oxoéthyle, préparé suivant l'exemple 5, et de 0,074 g (0,389 mmoles) d'acide 4-trifluorométhylphenylboronique, on obtient 0,049 g de produit sous forme de poudre.
Point de fusion (°C) : 197-199
LC-MS : M+H = 451
RMN-¹H (DMSO) δ (ppm) : 7,85-7,65 (m, 7H), 7, 05 (d, 2H), 4,35 (s,2H), 4,05 (d large,2H), 3,90 (d,2H), 2,85 (m,2H), 2,60 (d,3H), 2,00 (m,1H), 1,80 (d large,2H), 1,35-1, 10 (m, 2H).

### Exemple 7 (composé n° 137)

### 4-[(1-naphtalènyloxy)méthyl]-1-pipéridinecarboxylate de 2-amino-2-oxoéthyle

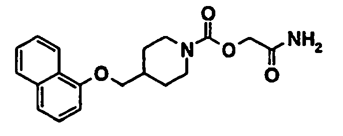

### 7.1 4-[(1-naphtalènyloxy)méthyl]-1-pipéridinecarboxylate de 1,1-diméthyléthyle

A une solution de 5,0 g (23,2 mmoles) de 4-(hydroxyméthyl)-1-pipéridinecarboxylate de 1,1-diméthyléthyle, 4,3 g (29,8 mmoles) de 1-naphtalènol et 7,82 g (29,8 mmoles) de triphénylphosphine dans 120 ml de tétrahydrofurane, refroidie sous azote par un bain de glace, on ajoute goutte à goutte une solution de 6,03 g (29,8 mmoles) de azodicarboxylate de diisopropyle. On laisse le mélange réactionnel revenir à température ambiante et on poursuit l'agitation pendant une nuit. On ajoute 2 ml de méthanol puis on évapore à sec. On reprend le résidu dans 200 ml de dichlorométhane et on lave successivement par une solution aqueuse à 10% d'hydrogénosulfate de potassium, de l'eau et une solution aqueuse d'hydroxyde de sodium 1M. On sèche sur sulfate de sodium et on évapore à sec. On purifie le produit par chromatographie sur gel de silice en éluant par un mélange 80/20 puis 70/30 et 50/50 de cyclohexane et de dichlorométhane pour obtenir 7,96 g de produit sous forme d'huile qui se solidifie.
Point de fusion (°C) : 97-100

### 7.2. 4-[(1-naphtalènyloxy)méthyl]pipéridine

On chauffe à 60°C pendant 6 heures une solution de 7,96 g (29,1 mmoles) de 4-[(1-naphtalènyloxy)méthyl]-1-pipéridinecarboxylate de 1,1-diméthyléthyle, préparé à l'étape 7.1., dans 120 ml de méthanol et 28 ml d'acide chlorhydrique aqueux à 35%. On refroidit à température ambiante et on évapore à sec puis on coévapore 2 fois avec de l'éthanol. On lave le résidu solide par du diéthyléther puis on sèche sous vide en présence de pentoxyde de phosphore pour obtenir 3,1 g de solide blanc.
On reprend le solide dans 80 ml d'eau et on ajoute une solution aqueuse d'hydroxyde de sodium à 30% jusqu'à pH basique puis on extrait 2 fois par 150 ml de diéthyléther. On sèche les extraits sur sulfate de sodium et on concentre à sec pour obtenir 2,75 g de produit huileux utilisé tel quel dans l'étape suivante.

### 7.3. 4-[(1-naphtalènyloxy)méthyl]-1-pipéridinecarboxylate de 2-(éthyloxy) -2-oxoéthyle

On chauffe à 50°C pendant une nuit un solution de 2,75 g (11,4 mmol es) de 4-[(1-naphtalènyloxy)méthyl]pipéridine, préparé à l'étape 7.2., et de 2,56 g (11,4 mmoles) de [(phényloxycarbonyl)oxy]acétate d'éthyle, préparé suivant l'exemple 1.1, dans 80 ml de toluène. On évapore à sec et on reprend le résidu par un mélange d'eau, de dichlorométhane et d'une solution aqueuse saturée en hydrogénocarbonate de sodium. On décante la phase organique, on la sèche sur sulfate de sodium et on l'évapore à sec. On purifie le produit par chromatographie sur gel de silice en éluant par un mélange 50/50 de cyclohexane et de dichlorométhane puis par du dichlorométhane et par un mélange 95/5 de dichlorométhane et d'acétate d'éthyle. On obtient 2,05 g de produit sous forme d'huile utilisé tel quel dans l'étape suivante.

### 7.4. 4-[(1-naphtalènyloxy)méthyl]-1-pipéridinecarboxylate de 2-amino-2-oxoéthyle

On dissout 1,0 g (2,69 mmoles) de 4-[(1-naphtalènyloxy)méthyl]-1-pipéridinecarboxylate de 2-(éthyloxy)-2-oxoéthyle, préparé à l'étape 7.3., dans 12 ml d'une solution d'ammoniac 7N (84 mmoles) dans le méthanol. On laisse réagir à température ambiante pendant 3 jours. On évapore à sec et on purifie par chromatographie sur gel de silice en éluant par un mélange 90/10 puis 80/20, 70/30 et 50/50 de dichlorométhane et d'acétate d'éthyle puis par un mélange 95/5 d'acétate d'éthyle et de méthanol. On recristallise ensuite dans l'acétate d'éthyle, pour obtenir 0,77 g de produit.
Point de fusion (°C) . 135-136
LC-MS : M+H = 343
RMN-¹H (DMSO) δ (ppm) : 8, 15 (dd, 1H), 7, 80 (dd, 1H), 7,50-7,30 (m,4H), 7,30 (m, 1H), 7,15 (m, 1H), 6,95 (d, 1H), 4,35 (s,2H), 4,15-4,00 (m+d,4H), 4,90 (m,2H), 2,10 (m, 1H), 1,90 (d,2H), 1,45,-1,25 (m,2H)

### Exemple 8 (composé n° 148)

### 4-[(7-quinolinyloxy)méthyl]-1-pipéridinecarboxylate de 2-amino-2-oxoéthyle

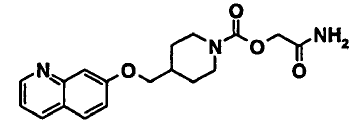

### 8.1. 4-(hydroxyméthyl)-1-piperidinecarboxylate de 2-(méthyloxy)-2-oxoéthyle

On procède comme décrit dans l'exemple 2.1., en utilisant 6,84 g (59,4 mmoles) de 4-(hydroxyméthyl)pipéridine, au lieu de l'éthanolamine, pour obtenir 7,85 g de produit sous forme d'huile incolore.

### 8.2. 4-[(7-quinolinyloxy)méthyl]-1-pipéridinecarboxylate de 2-amino-2-oxoéthyle

On ajoute 0,26 g (1,03 mmoles) de 1,1'-(azodicarbonyl)dipipéridine (ADDP) à une solution de 0,16 g (0,69 mmoles) de 4-(hydroxyméthyl)-1-piperidinecarboxylate de 2-(méthyloxy)-2-oxoéthyle, préparé à l'étape 8.1., de 0,26 ml (1,03 mmoles) de tri-n-butylphosphine et de 0,13 g (0,90 mmoles) de 7-hydroxyquinoline dans 2,5 ml de benzène refroidie par un bain de glace. On agite le mélange pendant 15 minutes à 0°C puis à température ambiante pendant 16 heures.

On filtre sur célite et on rince par du diéthyléther. On évapore à sec les filtrats et on purifie par chromatographie sur gel de silice en éluant par un mélange 70/30 d'acétate d'éthyle et de n-hexane. On dissout le produit obtenu dans 3 ml (21 mmoles) d'une solution d'ammoniac 7M dans le méthanol. On agite pendant 3 heures puis on évapore à sec. On purifie le produit par chromatographie sur gel de silice en éluant par un mélange 90/10 d' acétate d'éthyle et d'éthanol et on recristallise dans l'acétate d'éthyle pour obtenir 0,115 g de produit sous forme de solide blanc.
Point de fusion (°C) : 137-139
LC-MS : M+H = 344
RMN-¹H (CDCl₃) δ (ppm) : 7,80 (dd, 1H), 8,05 (dd, 1H), 7,70 (d, 1H), 7,40 (d, 1H), 7,30-7,15 (m,2H), 6,05 (m, 1H), 5,65 (m, 1H), 4,60 (s, 2H), 4,25 (m, 2H), 4,00 (d, 2H), 2,90 (m,2H), 2,10 (m, 1H), 1,95 (d, 2H), 1,50-1,30 (m,2H)

### Exemple 9 (composé n° 168)

### 4-{2-[(4-bromophényl)oxy]éthyl}-1-pipéridinecarboxylate de 2-(méthylamino)-2-oxoéthyle

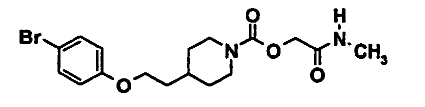

### 9.1. 4-{2-[(4-bromophényl)oxy]éthyl}-1-pipéridinecarboxylate de 1,1-diméthyléthyle

On procède comme décrit à l'exemple 4.1. A partir de 1,93 g (8,4 mmoles) de 4-(2-hydroxyéthyl)-1-pipéridinecarboxylate de 1,1-diméthyléthyle et de 5,88 g (33,6 mmoles) de 1-bromo-4-fluorobenzène, on obtient 4,1 g de produit brut sous forme d'huile.

### 9.2. 4-{2-[(4-bromophényl)oxy]ethyl}pipéridine

On procède comme décrit à l'exemple 4.2. A partir de 4-{2-[(4-bromophênyl)oxy]éthyl}-1-pipéridinecarboxylate de 1,1-diméthyléthyle, préparé à l'étape 9.1, on obtient 1,79 g de produit sous forme de poudre.
Point de fusion (°C) : 100-102

### 9.3. 4-{2-[(4-bromophényl)oxy]éthyl}-1-pipéridinecarboxylate de 2-(éthyloxy)-2-oxoéthyle

On procède comme décrit à l'exemple 4.3. A partir de 1,76 g (6,19 mmoles) de 4-{2- [(4-bromophényl)oxy]éthyl} pipéridine, préparé à l'étape 9.2, et de 1,39 g (6,19 mmoles) de {[(phényloxy)carbonyl]oxy}acétate d'éthyle, préparé suivant l'exemple 1.1, on obtient 1,4 g de produit sous forme d'huile.

### 9.4. 4-{2-[(4-bromophényl)oxy]éthyl}-1-pipéridinecarboxylate de 2-(méthylamino)-2-oxoéthyle

On procède comme décrit à l'exemple 4.4. A partir de 1,3 g (3,14 mmoles) de 4-{2-[(4-bromophényl)oxy]éthyl}-1-pipéridinecarboxylate de 2-(éthyloxy)-2-oxoéthyle, préparé à l'étape 9.3, on obtient 0,95 g de produit sous forme de poudre.
Point de fusion (°C) : 101 - 103
LC-MS : M+H = 400
RMN-¹H (CDCl₃) δ (ppm) : 7,55 (d, 2H) ; 7,00 (d, 2H) ; 6,25 (s large, NH); 4,90 - 4,70 (m, 2H); 4,50 - 4,25 (m, 2H); 4,20 (t, 2H); 3, 20 - 2,90 (m, 2H) ; 3, 10 (d, 3H) ; 2,05 - 1,90 (m, 5H) ; 1, 55 - 1,30 (m, 2H)

### Exemple 10 (composé n° 186)

### 4-{2-[(4'-chloro-4-biphényl)oxy]éthyl}-1-pipéridinecarboxylate de 2-(méthylamino)-2-oxoéthyle

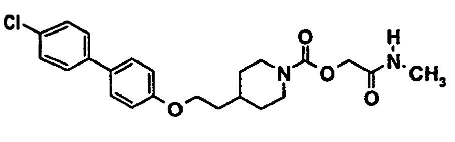

On procède comme décrit à l'exemple 4.5. A partir de 0,1 g (0,25 mmoles) de 4-{2-[(4-bromophényl)oxy]éthyl}-1-pipéridinecarboxylate de 2-(méthylamino)-2-oxoéthyle, préparé suivant l'exemple 9, et de 0,117 g (0,75 mmoles) d'acide 4-chlorophénylboronique, on obtient 0,087 g de produit sous forme de poudre.
Point de fusion (°C) : 104-106
LC-MS : M+H = 431
RMN-¹H (CDCl₃) δ (ppm) : 7,70 - 7,50 (m, 6H) ; 7,10 (d, 2H); 6,20 (s large, NH) ; 4,85 - 4,60 (m, 2H) ; 4,45 - 4,15 (m, 2H) ; 4,20 (t, 2H); 3,15 - 2,95 (m, 2H); 3,05 (d, 3H) ; 2,10 - 1,85 (m, 5H), 1,50 - 1,25 (m, 2H)

### Exemple 11 (composé n° 183)

### 4- [2- (7-isoquinolinyloxy) éthyl] -1-pipéridinecarbamate de 2-amino-2-oxoéthyle

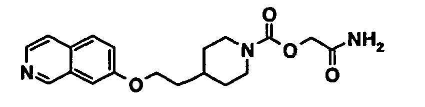

### 11.1. 4-(2-hydroxyéthyl)-1-pipéridinecarpoxylate de 2-(méthyloxy)-2-oxoéthyle

On procède comme décrit dans l'exemple 2.1., en utilisant 7,6 g (59,4 mmoles) de 4-(2-hydroxyéthyl)pipéridine, au lieu de l'éthanolamine, pour obtenir 7,1 g de produit sous forme d'huile incolore.

### 11.2. 4-[2-(7-isoquinolinyloxy)éthyl]-1-pipéridinecarbamate de 2-amino-2-oxoéthyle

On procède comme décrit dans l'exemple 8.2., à partir de 0,46 g (1,84 mmoles) de ADDP, de 0,30 g (1,24 mmoles) de 4-(2-hydroxyéthyl)-1-pipéridinecarboxylate de 2-(méthyloxy)-2-oxoéthyle, préparé à l'étape 11.1., de 0,46 ml de tri-n-butylphosphine et de 0,26 g (1,84 mmoles) de 7-hydroxyisoquinoline dans 4 ml de benzène. On purifie le produit par chromatographie sur gel de silice en éluant par de l'acétate d'éthyle puis par un mélange 95/5 d'acétate d'éthyle et d'éthanol pour obtenir 0,25 g de produit sous forme de solide blanc.
Point de fusion (°C) : 179-181
LC-MS : M+H = 358
RMN-¹H (CDCl₃) δ (ppm) : 9,15 (s, 1H), 8,45 (d, 1H), 7,60 (d, 1H), 7,35 (dd, 1H), 7,20 (d, 1H), 6,05 (m, 1H), 5,75 (m, 1H), 4,60 (s,2H), 4,20 (t,4H), 2,90 (m,2H), 1,90-1,70 (m, 5H), 1, 40-1, 20 (m,2H)

### Exemple 12 (composé n° 83)

### 3-[(1-naphtalènyloxy)méthyl]-1- pyrrolidinecarboxylate de 2-amino-2-oxoéthyle

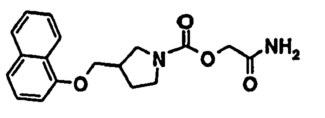

### 12.1. 3-[(1-naphtalènyloxy)méthyl]-1-pyrrolidinecarboxylate de 1,1-diméthyléthyle

A une solution de 1,0 g (4,9 mmoles) de 3-(hydroxyméthyl)-1-pyrrolidinecarboxylate de 1,1-diméthyléthyle (décrit dans WO0066557), 0,95 g (6,4 mmoles) de 1-naphtalènol et 1,4 g (6,9 mmoles) de tri-n-butylphosphine dans 40 ml de toluène et 20 ml de tétrahydrofurane, refroidie sous azote par un bain de glace, on ajoute goutte à goutte une solution de 1,74 g (6,9 mmoles) de ADDP. On laisse le mélange réactionnel revenir à température ambiante et on poursuit l'agitation pendant 24 heures. On filtre le mélange et on rince le précipité avec du toluène. On évapore à sec. On reprend le résidu dans du dichlorométhane et on lave par une solution aqueuse d'hydroxyde de sodium 1M. On sèche sur sulfate de sodium et on évapore à sec. On purifie le résidu par chromatographie sur colonne de gel de silice en éluant par du dichlorométhane puis un mélange 98/2 de dichlorométhane et de méthanol pour obtenir 0,80 g de produit sous forme d'huile incolore.

### 12.2. 3-[(1-naphtalènyloxy)méthyl]pyrrolidine

On agite pendant 6 heures une solution de 0,42 g (1,28 mmoles) de 3-[(1-naphtalènyloxy)méthyl]-1-pyrrolidinecarboxylate de 1,1-diméthyléthyle, préparé à l'étape 12.1., dans 10 ml de 1,4-dioxanne et 6 ml d'une solution 2N d'acide chlorhydrique aqueux. On évapore à sec puis on coévapore 2 fois avec du toluène. On lave le résidu solide par du diéthyléther. On reprend le solide dans du dichlorométhane et on ajoute une solution d'ammoniaque concentrée jusqu'à pH basique. On filtre sur cartouche Whatman PTFE et on concentre la phase organique pour obtenir 0,21 g de produit huileux utilisé tel quel dans l'étape suivante.

### 12.3. 3-[(1-naphtalènyloxy)méthyl]-1-pyrrolidinecarboxylate de 2-(éthyloxy)-2-oxoéthyle

On chauffe à 60°C pendant une nuit une solution de 0,20 g (0,88 mmoles) de 3-[(1-naphtalènyloxy)méthyl]pyrrolidine, préparé à l'étape 12.2., et de 0,35 g (1,5 mmoles) de [(phényloxycarbonyl)oxy]acétate d'éthyle, préparé suivant l'exemple 1.1, dans 6 ml de toluène. On évapore à sec et on reprend le résidu par un mélange d'eau, de dichlorométhane et d'une solution aqueuse saturée en hydrogénocarbonate de sodium. On décante la phase organique, on la sèche sur sulfate de sodium et on l'évapore à sec. On purifie le résidu par chromatographie sur colonne de gel de silice en éluant par du dichlorométhane puis par un mélange 99/1 de dichlorométhane et de méthanol. On obtient 0,24 g de produit sous forme d'huile utilisé tel quel dans l'étape suivante.

### 12.4. 3-[(1-naphtalènyloxy)méthyl]-1-pyrrolidinecarboxylate de 2-amino-2-oxoéthyle

On dissout 0,24 g (0,67 mmoles) de 3-[(1-naphtalènyloxy)méthyl]-1-pyrrolidinecarboxylate de 2-(éthyloxy)-2-oxoéthyle, préparé à l'étape 12.3., dans 15 ml d'une solution d'ammoniac 7N (105 mmoles) dans le méthanol. On agite en tube bouché à température ambiante pendant 3 jours. On évapore à sec et on purifie le résidu par chromatographie sur colonne de gel de silice en éluant par un mélange 97/3 puis 94/6 de dichlorométhane et de méthanol. On triture le solide obtenu dans du diéthyléther et on le filtre pour obtenir 0,15 g de produit.
Point de fusion (°C) : 161-163
LC-MS : M+H = 329
RMN-¹H (DMSO) δ (ppm) : 8, 15 (m, 1H), 7,75 (m, 1H), 7,50-7,30 (m,4H), 7,10-6,90 (s,2H), 6,80 (m, 1H), 4,40 (s, 2H), 4,20-4,05 (m,2H), 3,90-3,30 (m,4H), 2,90-2,70 (m, 1H), 2,30-2,10 (m, 1H), 2,05-1,85 (m, 1H).

Le tableau qui suit illustre les structures chimiques et les propriétés physiques de quelques composés selon l'invention.

### Tableau

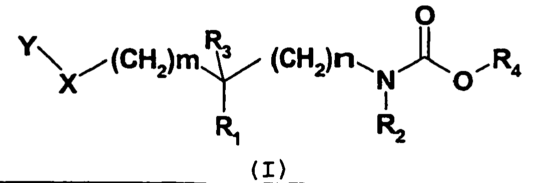

| **n°** | **Y** | **X** | **m** | **n** | **R₁** | **R₂** | **R₃** | **R₄** | **PF°C (ou M+H)** |
|---|---|---|---|---|---|---|---|---|---|
| 1. | 4-chlorophényl | O | 0 | 1 | H | H | H | CH₂CONHCH₃ | 147-149 |
| 2. | 4-chlorophényl | O | 0 | 1 | H | H | H | CH₂CONH₂ | 128-130 |
| 3. | 4-chlorophényl | O | 0 | 2 | H | H | H | CH₂CONH₂ | 108-110 |
| 4. | 4-chlorophényl | O | 0 | 3 | H | H | H | CH₂CONH₂ | 116-118 |
| 5. | 3-chlorophényl | O | 0 | 1 | H | H | H | CH₂CONH₂ | 114-116 |
| 6. | 3-chlorophényl | O | 0 | 2 | H | H | H | CH₂CONH₂ | 90-92 |
| 7. | 3-chlorophényl | O | 0 | 3 | H | H | H | CH₂CONH₂ | 114-116 |
| 8. | 2-chlorophényl | O | 0 | 1 | H | H | H | CH₂CONH₂ | 128-130 |
| 9. | 2-chlorophényl | O | 0 | 2 | H | H | H | CH₂CONH₂ | 128-130 |
| 10. | 2-chlorophényl | O | 0 | 3 | H | H | H | CH₂CONH₂ | 110-112 |
| 11. | 4-cyanophényl | O | 0 | 1 | H | H | H | CH₂CONH₂ | 204-206 |
| 12. | 4-cyanophényl | O | 0 | 2 | H | H | H | CH₂CONH₂ | 173-175 |
| 13. | 4-cyanophényl | O | 0 | 3 | H | H | H | CH₂CONH₂ | 169-171 |
| 14. | 3-cyanophényl | O | 0 | 1 | H | H | H | CH₂CONH₂ | 142-143 |
| 15. | 3-cyanophényl | O | 0 | 2 | H | H | H | CH₂CONH₂ | 129-131 |
| 16. | 3-cyanophényl | O | 0 | 3 | H | H | H | CH₂CONH₂ | 123-125 |
| 17. | 4-*iso*-propylphényl | O | 0 | 1 | H | H | H | CH₂CONH₂ | 95-97 |
| 18. | 4-*iso*-propylphényl | O | 0 | 2 | H | H | H | CH₂CONH₂ | (295) |
| 19. | 4-*iso*-propylphényl | O | 0 | 3 | H | H | H | CH₂CONH₂ | 103-105 |
| 20. | 3-*iso*-propylphényl | O | 0 | 1 | H | H | H | CH₂CONH₂ | 96-98 |
| 21. | 3-*iso*-propylphényl | O | 0 | 2 | H | H | H | CH₂CONH₂ | (295) |
| 22. | 3-*iso*-propylphényl | O | 0 | 3 | H | H | H | CH₂CONH₂ | 88-90 |
| 23. | 4-*tert*-butylphényl | O | 0 | 2 | H | H | H | CH₂CONH₂ | 90-92 |
| 24. | 4-*tert*-butylphényl | O | 0 | 3 | H | H | H | CH₂CONH₂ | (323) |
| 25. | 3-*tert*-butylphényl | O | 0 | 2 | H | H | H | CH₂CONH₂ | (309) |
| 26. | 3-*tert*-butylphényl | O | 0 | 3 | H | H | H | CH₂CONH₂ | (323) |
| 27. | 4-(1,1,3,3-tetraméthylbutyl)phényl | O | 0 | 2 | H | H | H | CH₂CONH₂ | (365) |
| 28. | 4-(1,1,3,3-tetraméthylbuty)phényl | O | 0 | 3 | H | H | H | CH₂CONH₂ | (379) |
| 29. | 4-(1,1-diméthylphényl méthyl)phényl | O | 0 | 1 | H | H | H | CH₂CONH₂ | (357) |
| 30. | 4-(1,1-diméthylphényl méthyl)phényl | O | 0 | 2 | H | H | H | CH₂CONH₂ | (371) |
| 31. | 4-(1,1-diméthylphényl méthyl)phényl | O | 0 | 3 | H | H | H | CH₂CONH₂ | (385) |
| 32. | 4-phénylphényl | O | 0 | 1 | H | H | H | CH₂CONH₂ | 196-198 |
| 33. | 4-phénylphényl | O | 0 | 2 | H | H | H | CH₂CONH₂ | 187-189 |
| 34. | 4-phénylphényl | O | 0 | 3 | H | H | H | CH₂CONH₂ | 192-194 |
| 35. | 4-(4-chlorophényl)phényl | O | 0 | 1 | H | H | H | CH₂CONH₂ | 193-195 |
| 36. | 4-(3-chlorophényl)phényl | O | 0 | 1 | H | H | H | CH₂CONH₂ | 168-170 |
| 37. | 4-(2-chlorophényl)phényl | O | 0 | 1 | H | H | H | CH₂CONH₂ | 114-116 |
| 38. | 4-(4-méthoxyphényl) phényl | O | 0 | 1 | H | H | H | CH₂CONH₂ | 194-196 |
| 39. | 4-(3-méthoxyphényl) phényl | O | 0 | 1 | H | H | H | CH₂CONH₂ | (345) |
| 40. | 4-(2-méthoxyphényl) phényl | O | 0 | 1 | H | H | H | CH₂CONH₂ | 127-129 |
| 41. | 3-phénylphényl | O | 0 | 1 | H | H | H | CH₂CONH₂ | 126-128 |
| 42. | 3-phénylphényl | O | 0 | 2 | H | H | H | CH₂CONH₂ | 110-112 |
| 43. | 3-phénylphényl | O | 0 | 3 | H | H | H | CH₂CONH₂ | 127-129 |
| 44. | 3-(4-chlorophényl)phényl | O | 0 | 1 | H | H | H | CH₂CONH₂ | 137-139 |
| 45. | 3-(3-chlorophényl)phényl | O | 0 | 1 | H | H | H | CH₂CONH₂ | 90-92 |
| 46. | 3-(2-chlorophényl)phényl | O | 0 | 1 | H | H | H | CH₂CONH₂ | 55-57 |
| 47. | 3-(4-méthoxyphényl) phényl | O | 0 | 1 | H | H | H | CH₂CONH₂ | 168-170 |
| 48. | 3-(3-méthoxyphényl) phényl | O | 0 | 1 | H | H | H | CH₂CONH₂ | 86-88. |
| 49. | 2-phénylphényl | O | 0 | 1 | H | H | H | CH₂CONH₂ | 92-94 |
| 50. | 2-phénylphényl | O | 0 | 2 | H | H | H | CH₂CONH₂ | (329) |
| 51. | 2-phénylphényl | O | 0 | 3 | H | H | H | CH₂CONH₂ | (343) |
| 52. | 2-(4-chlorophényl)phényl | O | 0 | 1 | H | H | H | CH₂CONH₂ | 130-132 |
| 53. | 2-(3-chlorophényl)phényl | O | 0 | 1 | H | H | H | CH₂CONH₂ | 88-90 |
| 54. | 2-(2-chlorophényl)phényl | O | 0 | 1 | H | H | H | CH₂CONH₂ | (349) |
| 55. | 2-(4-méthoxyphényl) phényl | O | 0 | 1 | H | H | H | CH₂CONH₂ | 74-76 |
| 56. | naphthalèn-1-yl | O | 0 | 1 | H | H | H | CH₂CONH₂ | (289) |
| 57. | naphthalèn-1-yl | O | 0 | 2 | H | H | H | CH₂CONH₂ | (303) |
| 58. | naphthalèn-1-yl | O | 0 | 3 | H | H | H | CH₂CONH₂ | 80-82 |
| 59. | naphthalèn-1-yl | O | 0 | 3 | H | H | H | CH₂CONHCH₃ | 90-92 |
| 60. | 4-chloro-naphthalèn-1-yl | O | 0 | 3 | H | H | H | CH₂CONH₂ | 142-144 |
| 61. | 4-chloro-naphthalèn-1-yl | O | 0 | 3 | H | H | H | CH₂CONHCH₃ | 108-110 |
| 62. | naphthalèn-2-yl | O | 0 | 1 | H | H | H | CH₂CONH₂ | (289) |
| 63. | naphthalèn-2-yl | O | 0 | 2 | H | H | H | CH₂CONH₂ | 158-160 |
| 64. | naphthalèn-2-yl | O | 0 | 3 | H | H | H | CH₂CONH₂ | 171-173 |
| 65. | 4-phénoxyphényl | O | 0 | 1 | H | H | H | CH₂CONH₂ | 158-160 |
| 66. | 4-phénoxyphényl | O | 0 | 2 | H | H | H | CH₂CONH₂ | 141-143 |
| 67. | 4-phénoxyphényl | O | 0 | 3 | H | H | H | CH₂CONH₂ | 144-146 |
| 68. | pyridin-3-yl | O | 0 | 1 | H | H | H | CH₂CONH₂ | 135-137 |
| 69. | pyridin-3-yl | O | 0 | 2 | H | H | H | CH₂CONH₂ | 119-121 |
| 70. | pyridin-3-yl | O | 0 | 3 | H | H | H | CH₂CONH₂ | 96-98 |
| 71. | 5-chloroquinolin-8-yl | O | 0 | 1 | H | H | H | CH₂CONH₂ | 232-234 |
| 72. | 5-chloroquinolin-8-yl | O | 0 | 2 | H | H | H | CH₂CONH₂ | 183-185 |
| 73. | 5-chloroquinolin-8-yl | O | 0 | 3 | H | H | H | CH₂CONH₂ | 184-186 |
| 74. | phényl | O | 1 | 0 | H | H | CH₃ | CH₂CONH₂ | 90-92 |
| 75. | 4-CF₃-phényl | O | 0 | 2 | (CH₂)₂ | | H | CH₂CONH₂ | 115-117 |
| 76. | 4-Cl-phényl | SO₂ | 0 | 2 | (CH₂)₂ | | H | CH₂CONH₂ | 164-166 |
| 77. | 4-Cl-phényl | SO₂ | 0 | 2 | (CH₂)₂ | | H | CH₂CONHCH₃ | 168-170 |
| 78. | 3-CF₃-phényl | SO₂ | 0 | 2 | (CH₂)₂ | | H | CH₂CONH₂ | 148-150 |
| 79. | 3-CF₃-phényl | SO₂ | 0 | 2 | (CH₂)₂ | | H | CH₂CONHCH₃ | 163-165 |
| 80. | naphthalèn-1-yl | O | 0 | 1 | (CH₂)₂ | | H | CH₂CONHCH₃ | 111-113 |
| 81. | naphthalèn-1-yl | O | 1 | 1 | CH₂ | | H | CH₂CONH₂ | 176-178 |
| 82. | naphthalèn-1-yl | O | 1 | 1 | CH₂ | | H | CH₂CONHCH₃ | 112-114 |
| 83. | naphthalèn-1-yl | O | 1 | 1 | (CH₂)₂ | | H | CH₂CONH₂ | 161-163 |
| 84. | naphthalèn-1-yl | O | 1 | 1 | (CH₂)₂ | | H | CH₂CONHCH₃ | 99-101 |
| 85. | 4-(4-F-phényl)phényl | O | 0 | 2 | (CH₂)₂ | | H | CH₂CONHCH₃ | 117-119 |
| 86. | 4-(4-Cl-phényl)phényl | O | 0 | 2 | (CH₂)₂ | | H | CH₂CONHCH₃ | 134-136 |
| 87. | 4-(4-CH₃-phényl)phényl | O | 0 | 2 | (CH₂)₂ | | H | CH₂CONHCH₃ | 121-123 |
| 88. | 4-(4-*n*-butyl-phényl) phenyl | O | 0 | 2 | (CH₂)₂ | | H | CH₂CONHCH₃ | 105-107 |
| 89. | 4-(4-CF₃-phényl)phényl | O | 0 | 2 | (CH₂)₂ | | H | CH₂CONHCH₃ | 141-143 |
| 90. | 4-(4-CH₃O-phényl) phényl | O | 0 | 2 | (CH₂)₂ | | H | CH₂CONHCH₃ | 152-154 |
| 91. | 4-(4-C₂H₅O-phényl) phenyl | O | 0 | 2 | (CH₂)₂ | | H | CH₂CONHCH₃ | 145-147 |
| 92. | 4-(4-CF₃O-phényl)phényl | O | 0 | 2 | (CH₂)₂ | | H | CH₂CONHCH₃ | 131-133 |
| 93. | 4-(3-F,4-CH₃O-phényl) phenyl | O | 0 | 2 | (CH₂)₂ | | H | CH₂CONHCH₃ | (417) |
| 94. | 4-(3-Cl,4-F-phényl) phényl | O | 0 | 2 | (CH₂)₂ | | H | CH₂CONHCH₃ | 124-126 |
| 95. | 4-(3,4-Cl₂-phényl)phényl | O | 0 | 2 | (CH₂)₂ | | H | CH₂CONHCH₃ | (438) |
| 96. | 3-(4-F-phényl)phényl | O | 0 | 2 | (CH₂)₂ | | H | CH₂CONHCH₃ | (387) |
| 97. | 3-(4-Cl-phényl)phényl | O | 0 | 2 | (CH₂)₂ | | H | CH₂CONHCH₃ | (403) |
| 98. | 3-(4-CH₃-phényl)phényl | O | 0 | 2 | (CH₂)₂ | | H | CH₂CONHCH₃ | (383) |
| 99. | 3-(4-*n*-butyl-phényl) phenyl | O | 0 | 2 | (CH₂)₂ | | H | CH₂CONHCH₃ | (425) |
| 100 | 3-(4-CF₃-phényl)phényl | O | 0 | 2 | (CH₂)₂ | | H | CH₂CONHCH₃ | (437) |
| 101 | 3-(4-CH₃O-phényl) phényl | O | 0 | 2 | (CH₂)₂ | | H | CH₂CONHCH₃ | (399) |
| 102 | 3-(4-C₂H₅O-phényl) phenyl | O | 0 | 2 | (CH₂)₂ | | H | CH₂CONHCH₃ | (413) |
| 103 | 3-(4-CF₃O-phényl)phényl | O | 0 | 2 | (CH₂)₂ | | H | CH₂CONHCH₃ | (453) |
| 104 | 3-(3-F,4-CH₃O-phényl) phenyl | O | 0 | 2 | (CH₂)₂ | | H | CH₂CONHCH₃ | (417) |
| 105 | 3-(3-Cl,4-F-phényl) phényl | O | 0 | 2 | (CH₂)₂ | | H | CH₂CONHCH₃ | (421) |
| 106 | 3-(3,4-Cl₂-phényl)phényl | O | 0 | 2 | (CH₂)₂ | | H | CH₂CONHCH₃ | (438) |
| 107 | 3-(2,4-Cl₂-phényl)phényl | O | 0 | 2 | (CH₂)₂ | | H | CH₂CONHCH₃ | (438) |
| 108 | naphthalèn-1-yl | O | 0 | 2 | (CH₂)₂ | | H | CH₂CONH₂ | 137-138 |
| 109 | naphthalèn-1-yl | O | 0 | 2 | (CH₂)₂ | | H | CH₂CONHCH₃ | 121-122 |
| 110 | naphthalèn-2-yl | O | 0 | 2 | (CH₂)₂ | | H | CH₂CONH₂ | 118-120 |
| 111 | quinolin-8-yl | O | 0 | 2 | (CH₂)₂ | | H | CH₂CONH₂ | (330) |
| 112 | phényl | O | 1 | 2 | (CH₂)₂ | | H | CH₂CONH₂ | 123-125 |
| 113 | 4-Cl-phényl | O | 1 | 2 | (CH₂)₂ | | H | CH₂CONH₂. | 152-154 |
| 114 | 4-Cl-phényl | O | 1 | 2 | (CH₂)₂ | | H | CH₂CONHCH₃ | 166-168 |
| 115 | 4-Cl-phényl | S | 1 | 2 | (CH₂)₂ | | H | CH₂CONHCH₃ | 130-132 |
| 116 | 4-Cl-phényl | SO₂ | 1 | 2 | (CH₂)₂ | | H | CH₂CONHCH₃ | 131-133 |
| 117 | 3-Cl-phényl | O | 1 | 2 | (CH₂)₂ | | H | CH₂CONH₂ | 131-133 |
| 118 | 3-Cl-phényl | S | 1 | 2 | (CH₂)₂ | | H | CH₂CONHCH₃ | 94-96 |
| 119 | 3-Cl-phényl | SO₂ | 1 | 2 | (CH₂)₂ | | H | CH₂CONHCH₃ | 100-102 |
| 120 | 4-Br-phényl | O | 1 | 2 | (CH₂)₂ | | H | CH₂CONHCH₃ | 163-165 |
| 121 | 4-CH₃O-phényl | O | 1 | 2 | (CH₂)₂ | | H | CH₂CONH₂ | 128-130 |
| 122 | 3-CH₃O-phényl | O | 1 | 2 | (CH₂)₂ | | H | CH₂CONH₂ | 119-121 |
| 123 | 4-n-propyloxy-phényl | O | 1 | 2 | (CH₂)₂ | | H | CH₂CONH₂ | 141-143 |
| 124 | 4-CF₃-phényl | O | 1 | 2 | (CH₂)₂ | | H | CH₂CONH₂ | 134-136 |
| 125 | 4-CF₃-phényl | S | 1 | 2 | (CH₂)₂ | | H | CH₂CONHCH₃ | 129-131 |
| 126 | 4-CF₃-phényl | SO₂ | 1 | 2 | (CH₂)₂ | | H | CH₂CONHCH₃ | 103-105 |
| 127 | 3-CF₃-phényl | O | 1 | 2 | (CH₂)₂ | | H | CH₂CONH₂ | 120-121 |
| 128 | 3-CF₃-phényl | S | 1 | 2 | (CH₂)₂ | | H | CH₂CONHCH₃ | 167-169 |
| 129 | 3-CF₃-phényl | SO₂ | 1 | 2 | (CH₂)₂ | | H | CH₂CONHCH₃ | 141-143 |
| 130 | 4-CF₃O-phényl | O | 1 | 2 | (CH₂)₂ | | H | CH₂CONH₂ | 118-120 |
| 131 | 3-CF₃O-phényl | O | 1 | 2 | (CH₂)₂ | | H | CH₂CONH₂ | 132-134 |
| 132 | 4-isopropyl-phényl | O | 1 | 2 | (CH₂)₂ | | H | CH₂CONH₂ | 113-115 |
| 133 | 3-isopropyl-phényl | O | 1 | 2 | (CH₂)₂ | | H | CH₂CONH₂ | 119-121 |
| 134 | 2,3-Cl₂-phényl | O | 1 | 2 | (CH₂)₂ | | H | CH₂CONHCH₃ | 160-162 |
| 135 | 2,4-Cl₂-phényl | O | 1 | 2 | (CH₂)₂ | | H | CH₂CONHCH₃ | 144-146 |
| 136 | 3,4-Cl₂-phényl | O | 1 | 2 | (CH₂)₂ | | H | CH₂CONH₂ | 113-115 |
| 137 | naphthalèn-1-yl | O | 1 | 2 | (CH₂)₂ | | H | CH₂CONH₂ | 135-136 |
| 138 | naphthalèn-1-yl | O | 1 | 2 | (CH₂)₂ | | H | CH₂CONHCH₃ | 151-152 |
| 139 | naphthalèn-1-yl | O | 1 | 1 | (CH₂)₃ | | H | CH₂CONH₂ | 149-151 |
| 140 | naphthalèn-1-yl | O | 1 | 1 | (CH₂)₃ | | H | CH₂CONHCH₃ | (357) |
| 141 | naphthalèn-1-yl | S | 1 | 2 | (CH₂)₂ | | H | CH₂CONHCH₃ | 127-129 |
| 142 | naphthalèn-1-yl | SO₂ | 1 | 2 | (CH₂)₂ | | H | CH₂CONHCH₃ | 154-156 |
| 143 | naphthalèn-2-yl | O | 1 | 2 | (CH₂)₂ | | H | CH₂CONH₂ | 142-144 |
| 144 | naphthalèn-2-yl | S | 1 | 2 | (CH₂)₂ | | H | CH₂CONHCH₃ | 111-113 |
| 145 | naphthalèn-2-yl | SO₂ | 1 | 2 | (CH₂)₂ | | H | CH₂CONHCH₃ | 105-107 |
| 146 | 4-chloro-naphthalèn-1-yl | O | 1 | 2 | (CH₂)₂ | | H | CH₂CONH₂ | 166-168 |
| 147 | 4-chloro-naphthalèn-1-yl | O | 1 | 2 | (CH₂)₂ | | H | CH₂CONHCH₃ | 163-165 |
| 148 | quinolin-7-yl | O | 1 | 2 | (CH₂)₂ | | H | CH₂CONH₂ | 137-139 |
| 149 | isoquinolin-7-yl | O | 1 | 2 | (CH₂)₂ | | H | CH₂CONH₂ | 162-164 |
| 150 | 4-(4-F-phényl)phényl | O | 1 | 2 | (CH₂)₂ | | H | CH₂CONHCH₃ | 150-152 |
| 151 | 4-(4-Cl-phényl)phényl | O | 1 | 2 | (CH₂)₂ | | H | CH₂CONHCH₃ | 167-169 |
| 152 | 4-(4-CH₃-phényl)phényl | O | 1 | 2 | (CH₂)₂ | | H | CH₂CONHCH₃ | 164-166 |
| 153 | 4-(4-*n*-butyl-phényl) phenyl | O | 1 | 2 | (CH₂)₂ | | H | CH₂CONHCH₃ | 171-173 |
| 154 | 4-(4-CF₃-phényl)phényl | O | 1 | 2 | (CH₂)₂ | | H | CH₂CONHCH₃ | 197-199 |
| 155 | 4-(4-CH₃O-phényl) phényl | O | 1 | 2 | (CH₂)₂ | | H | CH₂CONHCH₃ | 172-174 |
| 156 | 4-(4-C₂H₅O-phényl)phényl | O | 1 | 2 | (CH₂)₂ | | H | CH₂CONHCH₃ | 173-175 |
| 157 | 4-(4-CF₃O-phényl)phényl | O | 1 | 2 | (CH₂)₂ | | H | CH₂CONHCH₃ | 152-154 |
| 158 | 4-(3-F,4-CH₃O-phényl) phenyl | O | 1 | 2 | (CH₂)₂ | | H | CH₂CONHCH₃ | 156-158 |
| 159 | 4-(3-Cl,4-F-phényl) phényl | O | 1 | 2 | (CH₂)₂ | | H | CH₂CONHCH₃ | 132-134 |
| 160 | 4-(3,4-Cl₂-phényl)phényl | O | 1 | 2 | (CH₂)₂ | | H | CH₂CONHCH₃ | 163-165 |
| 161 | 4-(2,4-Cl₂-phényl)phényl | O | 1 | 2 | (CH₂)₂ | | H | CH₂CONHCH₃ | 177-179 |
| 162 | pyrimidin-2-yl | S | 1 | 2 | (CH₂)₂ | | H | CH₂CONHCH₃ | 109-111 |
| 163 | 5-phényl-thiazol-2-yl | S | 1 | 2 | (CH₂)₂ | | H | CH₂CONHCH₃ | (406) |
| 164 | benzoxazol-2-yl | S | 1 | 2 | (CH₂)₂ | | H | CH₂CONHCH₃ | (364) |
| 165 | phenyl | O | 2 | 2 | (CH₂)₂ | | H | CH₂CONH₂ | 136-138 |
| 166 | 4-Cl-phényl | O | 2 | 2 | (CH₂)₂ | | H | CH₂CONH₂ | 115-117 |
| 167 | 3-Cl-phényl | O | 2 | 2 | (CH₂)₂ | | H | CH₂CONH₂ | 91-93 |
| 168 | 4-Br-phényl | O | 2 | 2 | (CH₂)₂ | | H | CH₂CONHCH₃ | 101-103 |
| 169 | 4-CH₃O-phényl | O | 2 | 2 | (CH₂)₂ | | H | CH₂CONH₂ | 108-110 |
| 170 | 3-CH₃O-phényl | O | 2 | 2 | (CH₂)₂ | | H | CH₂CONH₂ | (337) |
| 171 | 4-n-propyloxy-phényl | O | 2 | 2 | (CH₂)₂ | | H | CH₂CONH₂ | 121-123 |
| 172 | 4-CF₃-phényl | O | 2 | 2 | (CH₂)₂ | | H | CH₂CONH₂ | 112-114 |
| 173 | 3-CF₃-phényl | O | 2 | 2 | (CH₂)₂ | | H | CH₂CONH₂ | 94-96 |
| 174 | 4-CF₃O-phényl | O | 2 | 2 | (CH₂)₂ | | H | CH₂CONH₂ | 79-81 |
| 175 | 3-CF₃O-phényl | O | 2 | 2 | (CH₂)₂ | | H | CH₂CONH₂ | (391) |
| 176 | 4-isopropyl-phényl | O | 2 | 2 | (CH₂)₂ | | H | CH₂CONH₂ | 75-77 |
| 177 | 3-isopropyl-phényl | O | 2 | 2 | (CH₂)₂ | | H | CH₂CONH₂ | (349) |
| 178 | 3,4-Cl₂-phényl | O | 2 | 2 | (CH₂)₂ | | H | CH₂CONH₂ | 103-105 |
| 179 | naphthalèn-1-yl | O | 2 | 2 | (CH₂)₂ | | H | CH₂CONH₂ | 134-135 |
| 180 | naphthalèn-1-yl | O | 2 | 2 | (CH₂)₂ | | H | CH₂CONHCH₃ | 108-109 |
| 181 | naphthalèn-2-yl | O | 2 | 2 | (CH₂)₂ | | H | CH₂CONH₂ | (357) |
| 182 | quinolin-7-yl | O | 2 | 2 | (CH₂)₂ | | H | CH₂CONH₂ | 164-166 |
| 183 | isoquinolin-7-yl | O | 2 | 2 | (CH₂)₂ | | H | CH₂CONH₂ | 179-181 |
| 184 | 4-phényl-phényl | O | 2 | 2 | (CH₂)₂ | | H | CH₂CONHCH₃ | 124-126 |
| 185 | 4-(4-F-phényl)phényl | O | 2 | 2 | (CH₂)₂ | | H | CH₂CONHCH₃ | 120-122 |
| 186 | 4-(4-Cl-phényl)phényl | O | 2 | 2 | (CH₂)₂ | | H | CH₂CONHCH₃ | 104-106 |
| 187 | 4-(4-CF₃-phényl)phényl | O | 2 | 2 | (CH₂)₂ | | H | CH₂CONHCH₃ | 137-139 |
| 188 | 4-(3-CF₃-phényl)phényl | O | 2 | 2 | (CH₂)₂ | | H | CH₂CONHCH₃ | 98-100 |
| 189 | 4-(4-CH₃O-phényl) phényl | O | 2 | 2 | (CH₂)₂ | | H | CH₂CONHCH₃ | 141-143 |
| 190 | 4-(4-CF₃O-phényl)phényl | O | 2 | 2 | (CH₂)₂ | | H | CH₂CONHCH₃ | 116-118 |
| 191 | 4-(4-*iso*-propyl-phényl)phényl | O | 2 | 2 | (CH₂)₂ | | H | CH₂CONHCH₃ | 121-123 |
| 192 | 4 (2,4-Cl₂-phényl)phényl | O | 2 | 2 | (CH₂)₂ | | H | CH₂CONHCH₃ | 112-114 |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734). Ainsi, les cerveaux de souris (moins le cer-velet) sont prélevés et conservés à - 80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide. Dans ces conditions, les composés les plus actifs de l'invention présentent des CI₅₀ (concentration inhibant de 50% l'activité enzymatique contrôle de la FAAH) comprises entre 0,001 et 1 µM. Par exemple, le composé n°58 du tableau présente une CI₅₀ de 0,047 µM.

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9% contenant 5% d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale en suspension dans du Tween 80 à 0,5%, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg. Par exemple, le composé n°58 du tableau réduit de 51 % le nombre d'étirements induits par le PBQ, à une dose de 1 mg/kg à 2 heures.

L'enzyme FAAH (Chemistry and Physics of Lipids, (2000), 108, 107-121) catalyse l'hydrolyse des dérivés endogènes d'amides et d'esters de différents acides gras tels que la N-arachidonoyiéthanolamine (anandamide), la *N*-palmitoyl-éthanolamine, la *N*-oléoyl éthanolamine, l'oléamide ou le 2-arachidonoylglycérol. Ce s dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ ou tous autres substrats métabolisés par l'enzyme FAAH, sont impliqués.
On peut par exemple citer les maladies et les affections suivantes :
la douleur notamment les douleurs aiguës ou chroniques de type neurogène : migraine, douleurs neuropathiques incluant les formes associées au virus de l'herpès et au diabète ; les douleurs aiguës ou chroniques associées aux maladies inflammatoires: arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ; les maladies neuro-dégénératives aiguës et chroniques: maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et
tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes: psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires, virales ou bactériennes : SIDA, méningites ; les maladies inflammatoires, notamment les maladies articulaires : arthrite, arthrite rhumatoïde, ostéoarthrite, spondylite, goutte, vascularite, maladie de Crohn, syndrome du colon irritable ; l'ostéoporose ; les affections oculaires : hypertension oculaire, glaucome ;
les affections pulmonaires : maladies des voies respiratoires, bronchospasmes, toux, asthme, bronchite chronique, obstruction chronique des voies respiratoires, emphysème ;
les maladies gastro-intestinales : syndrome du colon irritable, désordres inflammatoires intestinaux, ulcères, diarrhées ;
l'incontinence urinaire et l'inflammation vésicale.

L'utilisation des composés de formule (I), à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé de formule (I). Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.
Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.
Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.
A titre d'exemple, une forme unitaire d'administration d'un composé selon l'invention sous forme de comprimé peut comprendre les composants suivants:

| | |
|---|---|
| Composé selon l'invention | 50,0 mg |
| Mannitol | 223,75 mg |
| Croscaramellose sodique | 6,0 mg |
| Amidon de mais | 15,0 mg |
| Hydroxypropyl méthylcellulose | 2,25 mg |
| Stéarate de magnésium | 3, 0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ces sels pharmaceutiquement acceptables, d'un solvat ou d'un hydrate dudit composé.

## Revendications

1. Composé répondant à la formule (I) 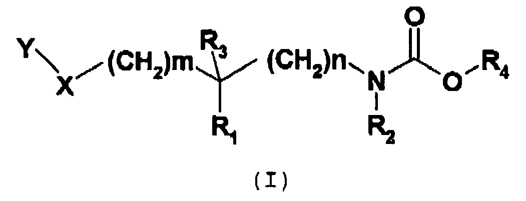 dans laquelle
m représente 0, 1, 2 ou 3 ;
n représente 0, 1, 2 ou 3 ;
X représente un atome d'oxygène ou de soufre ou un groupe SO ou SO₂ ;
R₁ et R₂ représentent, indépendamment l'un de l'autre, un atome d'hydrogène ou un groupe C₁₋₃-alkyle, ou R₁ et R₂ forment ensemble un groupe -(CH₂)ₚ-, où p représente un nombre entier allant de 1 à 5 tel que n + p soit un nombre entier allant de 2 à 5 ;
R₃ représente un atome d'hydrogène ou de fluor ou un groupe hydroxy ou méthyle ;
R₄ représente un groupe de formule générale CHR₅CONHR₆ dans laquelle
R₅ représente un atome d' hydrogène ou un groupe C₁₋₆-alkyle et R₆ représente un atome d'hydrogène ou un groupe C₁₋₆-alkyle, C₃₋₇-cycloalkyle, C₃₋₇-cycloalkyle-C₁₋₆-alkylène ;
Y représente
un groupe Y₁ choisi parmi notamment un phényle, pyridinyle, pyridazinyle, pyrimidinyle, pyrazinyle, triazinyle, thiazolyle, naphtyle, quinolinyle, isoquinolinyle, phtalazinyle, quinazolinyle, quinoxalinyle, naphtyridinyle, cinnolinyle, benzofuranyle, dihydrobenzofuranyle, benzo-thiényle, dihydrobenzothiényle, indolyle, isoindolyle, indolinyle, benzimidazolyle, benzoxazolyle, benzisoxazolyle, benzothiazolyle, benzisothiazolyle, benzotriazolyle, benzoxadiazolyle, benzothiadiazolyle ; le groupe Y₁ étant éventuellement substitué par un ou plusieurs substituants Y₂ identiques ou différents l'un de l'autre ou par un groupe Y₃ ; Y₂ représente un atome d'halogène, un groupe cyano, nitro, C₁₋₈-alkyle, C₁₋₈-alcoxy, C₁₋₈-thioalkyle, C₁₋₈-fluoroalkyle, C₁₋₈-fluoroalcoxy, C₁₋₈-fluorothioalkyle, C₃₋₇-cycloalkyle, C₃₋₇-cycloalkyloxy, C₃₋₇-cycloalkyle-C₁₋₈-alkylène, C₃₋₇-cycloalkyle-C₁₋₈-alkyloxy, hydroxy, NR₇R₈, NHCOR₇, NHSO₂R₇, COR₇, CO₂R₇, CONR₇R₈, SO₂R₇, SO₂NR₇R₈, -O-(C₁₋₃-alkylène)-O-, phényloxy, phénylthio, phényl-C₁-C₈-alkylène, phényl-C₁-C₈-alkyloxy ou phényl-C₁-C₈-alkylthio ; Y₃ représente un groupe choisi parmi notamment un phényle, pyridinyle, pyrimidinyle, pyrazinyle ou pyridazinyle ; le ou les groupes Y₃ pouvant être substitués par un ou plusieurs groupes Y₂ identiques ou différents l'un de l'autre ;
R₇ et R₈ représentent indépendamment l'un de l'autre un atome d'hydrogène ou un groupe C₁₋₆-alkyle, ou forment avec l'atome d'azote qui les porte un cycle azétidine, pyrrolidine, pipéridine, morpholine, thiomorpholine, azépine, pipérazine éventuellement substitué par un groupe C₁₋₃-alkyle ou benzyle ; à l'état de base, de sel d'addition à un acide, d'hydrate ou de solvat.

2. Composé de formule (I) selon la revendication 1,
**caractérisé en ce que**
Y représente
un groupe Y₁ choisi parmi notamment un phényle, pyridinyle, pyrimidinyle, thiazolyle, naphtyle, quinolinyle, isoquinolinyle, benzoxazolyle ; le groupe Y₁ étant éventuellement substitué par un ou plusieurs substituants Y₂ identiques ou différents l'un de l'autre ou par un groupe Y₃; Y₂ représente un atome d'halogène, un groupe cyano, C₁₋₈-alkyle, C₁₋₈-alcoxy, C₁₋₈-fluoroalkyle, C₁₋₈-fluoroalcoxy, phényloxy, phényl-C₁-C₈-alkylène ;
Y₃ représente un groupe phényle ; Y₃ pouvant être substitué par un ou plusieurs groupes Y₂ identiques ou différents l'un de l'autre ;
à l'état de base, de sel d'addition à un acide, d'hydrate ou de solvat.

3. Composé de formule (I) selon la revendication 1 ou 2,
**caractérisé en ce que**
Y représente
un groupe Y₁ choisi parmi notamment un phényle ou un naphtyle ; le groupe Y₁ étant éventuellement substitué par un ou plusieurs substituants Y₂ identiques ou différents l'un de l'autre ou par un groupe Y₃ ;
Y₂ représente un atome d'halogène, un groupe cyano, C₁₋₈-alkyle, C₁₋₈-alcoxy, C₁₋₈-fluoroalkyle, C₁₋₈-fluoroalcoxy, phényloxy, phényl-C₁-C₈-alkylène ;
Y₃ représente un groupe phényle ; Y₃ pouvant être substitué par un ou plusieurs groupes Y₂ identiques ou différents l'un de l'autre ;
à l'état de base, de sel d'addition à un acide, d'hydrate ou de solvat.

4. Composé de formule (I) selon l'une quelconque des revendications 1 à 3, **caractérisé en ce que**
m représente 0, 1, 2 ou 3 ;
n représente 0, 1, 2 ou 3 ;
R₁ et R₂ représentent, indépendamment l'un de l'autre, un atome d'hydrogène ou un groupe C₁₋₃-alkyle, ou R₁ et R₂ forment ensemble un groupe -(CH₂)ₚ-, où p représente un nombre entier allant de 1 à 5 tel que n + p soit un nombre entier allant de 2 à 5 ;
avec la condition que quand R₁ et R₂ représentent, indépendamment l'un de l'autre, un atome d'hydrogène ou un groupe C₁₋₃-alkyle, alors m + n > 1 ;
à l'état de base, de sel d'addition à un acide, d'hydrate ou de solvat.

5. Composé de formule (I) selon l'une quelconque des revendications 1 à 4, **caractérisé en ce que**
m représente 0, 1, 2 ou 3 ; et/ou
n représente 0, 1, 2 ou 3 ; et/ou
R₁ et R₂ forment ensemble un groupe -(CH₂)ₚ-, où p représente un nombre entier allant de 1 à 4 tel que n + p soit égal à 4 ; à l'état de base, de sel d'addition à un acide, d'hydrate ou de solvat.

6. Composé de formule (I) selon l'une quelconque des revendications 1 à 5, **caractérisé en ce que** X représente un atome d'oxygène ;
à l'état de base, de sel d'addition à un acide, d'hydrate ou de solvat.

7. Composé de formule (I) selon l'une quelconque des revendications 1 à 6, **caractérisé en ce que** R₃ représente un atome d'hydrogène;
à l'état de base, de sel d'addition à un acide, d'hydrate ou de solvat.

8. Composé de formule (I) selon la revendication 1 choisi parmi les composés suivants :
- {2-[(4-chlorophényl)oxy]éthyl}carbamate de 2-(méthylamino)-2-oxoéthyle ;
- (2-[(4-cyanophényl)oxy]éthyl)carbamate de 2-amino-2-oxoéthyle ;
- [4-(1-naphtalènyloxy]butyl]carbamate de 2-amino-2-oxoéthyle ;
- 4-[(4'-fluoro-4-biphényl)oxy]-1-pipéridinecarboxylate de 2-(méthylamino)-2-oxoéthyle;
- 4-{[(4-bromophényl)oxy]méthyl}-1-pipéridinecarboxylate de 2-(méthylamino)-2-oxoéthyle;
- 4-{[(4'-(trifluorométhyl)-4-biphényl)oxy]méthyl}-1-pipéridinecarboxylate de 2-(méthylamino)-2-oxoéthyle ;
- 4-[(1-naphtalènyloxy)méthyl]-1-pipéridinecarboxylate de 2-amino-2-oxoéthyle ;
- 4-[(7-quinolinyloxy)méthyl]-1-pipéridinecarboxylate de 2-amino-2-oxoéthyle ;
- 4-{2-[(4-bromophényl)oxy]éthyl}-1-pipéridinecarboxylate de 2-(méthylamino)-2-oxoéthyle ;
- 4-{2-[(4'-chloro-4-biphényl)oxy]éthyl}-1-pipéridinecarboxylate de 2-(méthylamino)-2-oxoéthyle ;
- 4-[2-(7-isoquinolinyloxy)éthyl]-1-pipéridinecarbamate de 2-amino-2-oxoéthyle ;
- 3-[(1-naphtalènyloxy)méthyl]-1-pyrrolidinecarboxylate de 2-amino-2-oxoéthyle.

9. Procédé de préparation d'un composé de formule (I) selon l'une quelconque des revendications 1 à 8, comprenant l'étape consistant à transformer un composé de formule (IV), 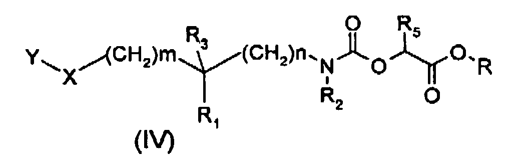 dans laquelle Y, X, R₁, R₂, R₃, R₅, m et n sont tels que définis selon l'une quelconque des revendications 1 à 8 et R représente un groupe méthyle ou éthyle,
par aminolyse au moyen d'une amine de formule R₆NH₂ où R₆ est tel que défini dans la formule (I) selon la revendication 1.

10. Composition pharmaceutique contenant au moins un composé de formule (I) selon l'une quelconque des revendications 1 à 8, à l'état de base, de sel, d'hydrate ou de solvat pharmaceutiquement acceptable et éventuellement un ou plusieurs excipients pharmaceutiquement acceptables.

11. Composé de formule (I) selon l'une quelconque des revendications 1 à 8, à l'état de base, de sel, d'hydrate ou de solvat pharmaceutiquement acceptable, pour son utilisation comme médicament.

12. Utilisation d'un composé de formule (I) selon l'une quelconque des revendications 1 à 8, à l'état de base, de sel, d'hydrate ou de solvat pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

13. Utilisation d'un composé de formule (I) selon l'une quelconque des revendications 1 à 8, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

## Claims

1. Compound conforming to the formula (I) 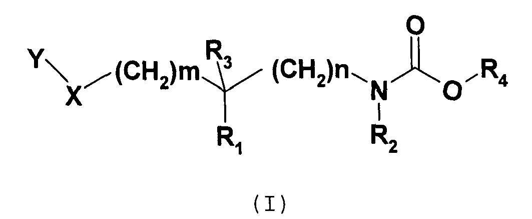 in which
m represents 0, 1, 2 or 3;
n represents 0, 1, 2 or 3;
X represents an oxygen or sulphur atom or an SO or SO₂ group;
R₁ and R₂ represent independently of one another a hydrogen atom or a C₁₋₃ alkyl group, or R₁ and R₂ together form a group -(CH₂)ₚ-, where p represents an integer ranging from 1 to 5 such that n + p is an integer ranging from 2 to 5;
R₃ represents a hydrogen or fluorine atom or a hydroxyl or methyl group;
R₄ represents a group of general formula CHR₅CONHR₆ in which
R₅ represents a hydrogen atom or a C₁₋₆ alkyl group and
R₆ represents a hydrogen atom or a C₁₋₆ alkyl, C₃₋₇ cycloalkyl or C₃₋₇ cycloalkyl-C₁₋₆ alkylene group;
Y represents
a group Y₁ selected from in particular a phenyl, pyridinyl, pyridazinyl, pyrimidinyl, pyrazinyl, triazinyl, thiazolyl, naphthyl, quinolinyl, isoquinolinyl, phthalazinyl, quinazolinyl, quinoxalinyl, naphthyridinyl, cinnolinyl, benzofuranyl, dihydrobenzofuranyl, benzothienyl, dihydrobenzothienyl, indolyl, isoindolyl, indolinyl, benzimidazolyl, benzoxazolyl, benzisoxazolyl, benzothiazolyl, benzisothiazolyl, benzotriazolyl, benzoxadiazolyl and benzothiadiazolyl; the group Y₁ being optionally substituted by one or more substituents Y₂, which are identical to or different from one another, or by a group Y₃;
Y₂ represents a halogen atom or a cyano, nitro, C₁₋₈ alkyl, C₁₋₈ alkoxy, C₁₋₈ thioalkyl, C₁₋₈ fluoroalkyl, C₁₋₈ fluoroalkoxy, C₁₋₈ fluorothioalkyl, C₃₋₇ cycloalkyl, C₃₋₇ cycloalkyloxy, C₃₋₇ cycloalkyl-C₁₋₈ alkylene, C₃₋₇ cycloalkyl-C₁₋₈ alkyloxy, hydroxyl, NR₇R₈, NHCOR₇, NHSO₂R₇, COR₇, CO₂R₇, CONR₇R₈, SO₂R₇, SO₂NR₇R₈, -O-(C₁₋₃ alkylene)-O-, phenyloxy, phenylthio, phenyl-C₁-C₈ alkylene, phenyl-C₁-C₈ alkyloxy or phenyl-C₁-C₈ alkylthio group;
Y₃ represents a group selected from in particular a phenyl, pyridinyl, pyrimidinyl, pyrazinyl or pyridazinyl; it being possible for the group or groups Y₃ to be substituted by one or more groups Y₂ which are identical to or different from one another;
R₇ and R₈ represent independently of one another a hydrogen atom or a C₁₋₆ alkyl group, or with the nitrogen atom carrying them form an azetidine, pyrrolidine, piperidine, morpholine, thiomorpholine, azepine or piperazine ring optionally substituted by a C₁₋₃ alkyl or benzyl group;
in the form of a base, addition salt with an acid, hydrate or solvate.

2. Compound of formula (I) according to Claim 1,
**characterized in that**
Y represents
a group Y₁ selected from in particular a phenyl, pyridinyl, pyrimidinyl, thiazolyl, naphthyl, quinolinyl, isoquinolinyl and benzoxazolyl; the group Y₁ being optionally substituted by one or more substituents Y₂, which are identical to or different from one another, or by a group Y₃;
Y₂ represents a halogen atom, a cyano, C₁₋₈ alkyl, C₁₋₈ alkoxy, C₁₋₈ fluoroalkyl, C₁₋₈ fluoroalkoxy, phenyloxy or phenyl-C₁-C₈ alkylene group;
Y₃ represents a phenyl group; it being possible for Y₃ to be substituted by one or more groups Y₂ which are identical to or different from one another;
in the form of a base, addition salt with an acid, hydrate or solvate.

3. Compound of formula (I) according to Claim 1 or 2,
**characterized in that**
Y represents
a group Y₁ selected from in particular a phenyl or a naphthyl; the group Y₁ being optionally substituted by one or more substituents Y₂, which are identical to or different from one another, or by a group Y₃;
Y₂ represents a halogen atom, a cyano, C₁₋₈ alkyl, C₁₋₈ alkoxy, C₁₋₈ fluoroalkyl, C₁₋₈ fluoroalkoxy, phenyloxy or phenyl-C₁-C₈ alkylene group;
Y₃ represents a phenyl group; it being possible for Y₃ to be substituted by one or more groups Y₂ which are identical to or different from one another;
in the form of a base, addition salt with an acid, hydrate or solvate.

4. Compound of formula (I) according to any one of Claims 1 to 3, **characterized in that**
m represents 0, 1, 2 or 3;
n represents 0, 1, 2 or 3;
R₁ and R₂ represent independently of one another a hydrogen atom or a C₁₋₃ alkyl group, or R₁ and R₂ together form a group -(CH₂)ₚ-, where p represents an integer ranging from 1 to 5 such that n + p is an integer ranging from 2 to 5;
with the proviso that, when R₁ and R₂ represent independently of one another a hydrogen atom or a C₁₋₃ alkyl group, m + n > 1;
in the form of a base, addition salt with an acid, hydrate or solvate.

5. Compound of formula (I) according to any one of Claims 1 to 4, **characterized in that**
m represents 0, 1, 2 or 3; and/or
n represents 0, 1, 2 or 3; and/or
R₁ and R₂ together form a group -(CH₂)ₚ-, where p represents an integer ranging from 1 to 4 such that n + p is equal to 4;
in the form of a base, addition salt with an acid, hydrate or solvate.

6. Compound of formula (I) according to any one of Claims 1 to 5, **characterized in that** X represents an oxygen atom;
in the form of a base, addition salt with an acid, hydrate or solvate.

7. Compound of formula (I) according to any one of Claims 1 to 6, **characterized in that** R₃ represents a hydrogen atom;
in the form of a base, addition salt with an acid, hydrate or solvate.

8. Compound of formula (I) according to Claim 1, selected from the following compounds:
- 2-(methylamino)-2-oxoethyl {2-[(4-chlorophenyl)oxy]ethyl}carbamate;
- 2-amino-2-oxoethyl (2-[(4-cyanophenyl)oxy]-ethyl)carbamate;
- 2-amino-2-oxoethyl [4-(1-naphthalenyloxy)butyl]carbamate;
- 2-(methylamino)-2-oxoethyl 4-[(4'-fluoro-4-biphenyl)oxy]-1-piperidinecarboxylate;
- 2-(methylamino)-2-oxoethyl 4-{[(4-bromophenyl)-oxy]methyl}-1-piperidinecarboxylate;
- 2-(methylamino)-2-oxoethyl 4-{[(4'-(trifluoromethyl)-4-biphenyl)oxy]methyl}-1-piperidine-carboxylate;
- 2-amino-2-oxoethyl 4-[(1-naphthalenyloxy)-methyl]-1-piperidinecarboxylate;
- 2-amino-2-oxoethyl 4-[(7-quinolinyloxy)methyl]-1-piperidinecarboxylate;
- 2-(methylamino)-2-oxoethyl 4-{2-[(4-bromophenyl)oxy]ethyl}-1-piperidinecarboxylate;
- 2-(methylamino)-2-oxoethyl 4-{2-[(4'-chloro-4-biphenyl)oxy]ethyl}-1-piperidinecarboxylate;
- 2-amino-2-oxoethyl 4-[2-(7-isoquinolinyloxy) ethyl]-1-piperidinecarbamate;
- 2-amino-2-oxoethyl 3-[(1-naphthalenyloxy)-methyl]-1-pyrrolidinecarboxylate.

9. Process for preparing a compound of formula (I) according to any one of Claims 1 to 8, comprising the step consisting in converting a compound of the formula (IV) 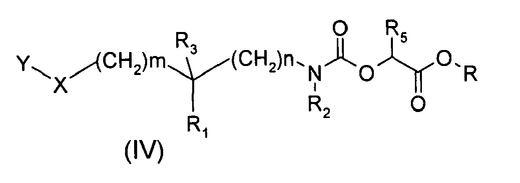 in which Y, X, R₁, R₂, R₃, R₅, n and m are as defined according to any one of Claims 1 to 8 and R represents a methyl or ethyl group, by aminolysis by means of an amine of formula R₆NH₂ where R₆ is as defined in formula (I) according to Claim 1.

10. Pharmaceutical composition comprising at least one compound of formula (I) according to any one of Claims 1 to 8, in base, salt, hydrate or solvate form that is pharmaceutically acceptable, and, optionally, one or more pharmaceutically acceptable excipients.

11. Compound of formula (I) according to any one of Claims 1 to 8, in base, salt, hydrate or pharmaceutically acceptable solvate form, for its use as a medicinal product.

12. Use of a compound of formula (I) according to any one of Claims 1 to 8, in base, salt, hydrate or pharmaceutically acceptable solvate form, for preparing a medicinal product intended for preventing or treating a pathology in which endogenous cannabinoids and/or any other substrates metabolized by the enzyme FAAH are involved.

13. Use of a compound of formula (I) according to any one of Claims 1 to 8, in base, salt, hydrate or pharmaceutically acceptable solvate form, for preparing a medicinal product intended for preventing or treating acute or chronic pain, dizziness, vomiting, nausea, eating disorders, neurological and psychiatric pathologies, acute or chronic neurodegenerative diseases, epilepsy, sleep disorders, cardiovascular diseases, renal ischemia, cancers, disorders of the immune system, allergic diseases, parasitic, viral or bacterial infectious diseases, inflammatory diseases, osteoporosis, ocular conditions, pulmonary conditions, gastrointestinal diseases or urinary incontinence.

## Patentansprüche

1. Verbindung der Formel (I) 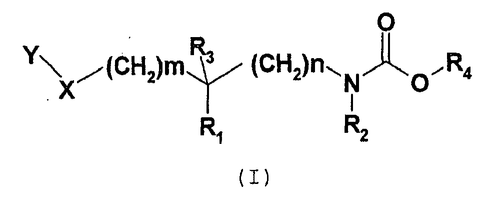 worin
m für 0, 1, 2 oder 3 steht;
n für 0, 1, 2 oder 3 steht;
X für ein Sauerstoff- oder Schwefelatom oder eine SO- oder SO₂-Gruppe steht;
R₁ und R₂ unabhängig voneinander für ein Wasserstoffatom oder eine C₁₋₃-Alkylgruppe stehen oder gemeinsam eine -(CH₂)ₚ-Gruppe bilden, worin p für eine solche ganze Zahl von 1 bis 5 steht, daß n + p für eine ganze Zahl von 2 bis 5 steht;
R₃ für ein Wasserstoff- oder Fluoratom oder eine Hydroxy- oder Methylgruppe steht;
R₄ für eine Gruppe der allgemeinen Formel CHR₅CONHR₆ steht, worin
R₅ für ein Wasserstoffatom oder eine C₁₋₆-Alkylgruppe steht und
R₆ für ein Wasserstoffatom oder eine C₁₋₆-Alkyl-, C₃₋₇-Cycloalkyl- oder C₃₋₇-Cycloalkyl-C₁₋₆-alkylengruppe steht;
Y für
eine Gruppe Y₁, insbesondere ausgewählt unter Phenyl, Pyridinyl, Pyridazinyl, Pyrimidinyl, Pyrazinyl, Triazinyl, Thiazolyl, Naphthyl, Chinolinyl, Isochinolinyl, Phthalazinyl, Chinazolinyl, Chinoxalinyl, Naphthyridinyl, Cinnolinyl, Benzofuranyl, Dihydrobenzofuranyl, Benzothienyl, Dihydrobenzothienyl, Indolyl, Isoindolyl, Indolinyl, Benzimidazolyl, Benzoxazolyl, Benzisoxazolyl, Benzothiazolyl, Benzisothiazolyl, Benzotriazolyl, Benzoxadiazolyl und Benzothiadiazolyl, steht; wobei die Gruppe Y₁ gegebenenfalls durch einen oder mehrere Substituenten Y₂, die gleich oder voneinander verschieden sind, oder durch eine Gruppe Y₃ substituiert ist;
Y₂ für ein Halogenatom oder eine Cyano-, Nitro-, C₁₋₈-Alkyl-, C₁₋₈-Alkoxy-, C₁₋₈-Thioalkyl-, C₁₋₈-Fluoralkyl-, C₁₋₈-Fluoralkoxy-, C₁₋₈-Fluorthioalkyl-, C₃₋₇-Cycloalkyl-, C₃₋₇-Cycloalkyloxy-, C₃₋₇-Cycloalkyl-C₁₋₈-alkylen-, C₃₋₇-Cycloalkyl-C₁₋₈-alkyloxy-, Hydroxy-, NR₇R₈-, NHCOR₇-, NHSO₂R₇-, COR₇-, CO₂R₇-, CONR₇R₈-, SO₂R₇-, SO₂NR₇R₈-, -O- (C₁₋₃-Alkylen)-O-, Phenyloxy-, Phenylthio-, Phenyl-C₁-C₈-alkylen-, Phenyl-C₁-C₈-alkyloxy- oder Phenyl-C₁-C₈-alkylthiogruppe steht;
Y₃ für eine insbesondere unter Phenyl, Pyridinyl, Pyrimidinyl, Pyrazinyl oder Pyridazinyl ausgewählte Gruppe steht;
wobei die Gruppe(n) Y₃ durch eine oder mehrere Gruppen Y₂, die gleich oder voneinander verschieden sind, substituiert sein kann bzw. können;
R₇ und R₈ unabhängig voneinander für ein Wasserstoffatom oder eine C₁₋₆-Alkylgruppe stehen oder mit dem Stickstoffatom, an das sie gebunden sind, einen Azetidin-, Pyrrolidin-, Piperidin-, Morpholin-, Thiomorpholin-, Azepin- oder Piperazinring bilden, der gegebenenfalls durch eine C₁₋₃-Alkyl- oder Benzylgruppe substituiert ist;
in Basen-, Säureadditionssalz-, Hydrat- oder Solvatform.

2. Verbindung der Formel (I) nach Anspruch 1, **dadurch gekennzeichnet, daß**
Y für
eine Gruppe Y₁, insbesondere ausgewählt unter Phenyl, Pyridinyl, Pyrimidinyl, Thiazolyl, Naphthyl, Chinolinyl, Isochinolinyl und Benzoxazolyl, steht; wobei die Gruppe Y₁ gegebenenfalls durch einen oder mehrere Substituenten Y₂, die gleich oder voneinander verschieden sind, oder durch eine Gruppe Y₃ substituiert ist;
Y₂ für ein Halogenatom oder eine Cyano-, C₁₋₈-Alkyl-, C₁₋₈-Alkoxy-, C₁₋₈-Fluoralkyl-, C₁₋₈-Fluoralkoxy-, Phenyloxy- oder Phenyl-C₁-C₈-alkylengruppe steht;
Y₃ für eine Phenylgruppe steht; wobei Y₃ durch eine oder mehrere Gruppen Y₂, die gleich oder voneinander verschieden sind, substituiert sein kann;
in Basen-, Säureadditionssalz-, Hydrat- oder Solvatform.

3. Verbindung der Formel (I) nach Anspruch 1 oder 2,
**dadurch gekennzeichnet, daß**
Y für
eine Gruppe Y₁, insbesondere ausgewählt unter Phenyl oder Naphthyl, steht; wobei die Gruppe Y₁ gegebenenfalls durch einen oder mehrere Substituenten Y₂, die gleich oder voneinander verschieden sind, oder durch eine Gruppe Y₃ substituiert ist;
Y₂ für ein Halogenatom oder eine Cyano-, C₁₋₈-Alkyl-, C₁₋₈-Alkoxy-, C₁₋₈-Fluoralkyl-, C₁₋₈-Fluoralkoxy-, Phenyloxy- oder Phenyl-C₁-C₈-alkylengruppe steht;
Y₃ für eine Phenylgruppe steht; wobei Y₃ durch eine oder mehrere Gruppen Y₂, die gleich oder voneinander verschieden sind, substituiert sein kann;
in Basen-, Säureadditionssalz-, Hydrat- oder Solvatform.

4. Verbindung der Formel (I) nach einem der Ansprüche 1 bis 3, **dadurch gekennzeichnet, daß**
m für 0, 1, 2 oder 3 steht;
n für 0, 1, 2 oder 3 steht;
R₁ und R₂ unabhängig voneinander für ein Wasserstoffatom oder eine C₁₋₃-Alkylgruppe stehen oder gemeinsam eine -(CH₂)ₚ-Gruppe bilden, worin p für eine solche ganze Zahl von 1 bis 5 steht, daß n + p für eine ganze Zahl von 2 bis 5 steht;
mit der Maßgabe, daß m + n > 1, wenn R₁ und R₂ unabhängig voneinander für ein Wasserstoffatom oder eine C₁₋₃-Alkylgruppe stehen;
in Basen-, Säureadditionssalz-, Hydrat- oder Solvatform.

5. Verbindung der Formel (I) nach einem der Ansprüche 1 bis 4, **dadurch gekennzeichnet, daß**
m für 0, 1, 2 oder 3 steht und/oder
n für 0, 1, 2 oder 3 steht und/oder
R₁ und R₂ gemeinsam eine -(CH₂)ₚ-Gruppe bilden, worin p für eine solche ganze Zahl von 1 bis 4 steht, daß n + p gleich 4 ist;
in Basen-, Säureadditionssalz-, Hydrat- oder Solvatform.

6. Verbindung der Formel (I) nach einem der Ansprüche 1 bis 5, **dadurch gekennzeichnet, daß** X für ein Sauerstoffatom steht;
in Basen-, Säureadditionssalz-, Hydrat- oder Solvatform.

7. Verbindung der Formel (I) nach einem der Ansprüche 1 bis 6, **dadurch gekennzeichnet, daß** R₃ für ein Wasserstoffatom steht;
in Basen-, Säureadditionssalz-, Hydrat- oder Solvatform.

8. Verbindung der Formel (I) nach Anspruch 1, ausgewählt unter den folgenden Verbindungen:
- {2-[(4-Chlorphenyl)oxy]ethyl}carbamidsäure-2-(methylamino)-2-oxoethylester;
- {2-[(4-Cyanophenyl)oxy]ethyl}carbamidsäure-2-amino-2-oxoethylester;
- [4-(1-Naphthalinyloxy)butyl]carbamidsäure-2-amino-2-oxoethylester;
- 4-[(4'-Fluor-4-biphenyl)oxy]-1-piperidincarbonsäure-2-(methylamino)-2-oxoethylester;
- 4-{[(4-Bromphenyl)oxy]methyl}-1-piperidincarbonsäure-2-(methylamino)-2-oxoethylester;
- 4-{[(4'-(Trifluormethyl)-4-biphenyl)oxy]methyl}-1-piperidincarbonsäure-2-(methylamino)-2-oxoethylester;
- 4-[(1-Naphthalinyloxy)methyl]-1-piperidincarbonsäure-2-amino-2-oxoethylester;
- 4-[(7-Chinolinyloxy)methyl]-1-piperidincarbonsäure-2-amino-2-oxoethylester;
- 4-{2-[(4-Bromphenyl)oxy]ethyl}-1-piperidin-carbonsäure-2-(methylamino)-2-oxoethylester;
- 4-{2-[(4'-Chlor-4-biphenyl)oxy]ethyl}-1-piperidincarbonsäure-2-(methylamino)-2-oxoethylester;
- 4-[2-(7-Isochinolinyloxy)ethyl]-1-piperidin-carbamidsäure-2-amino-2-oxoethylester;
- 3-[(1-Naphthalinyloxy)methyl]-1-pyrrolidin-carbonsäure-2-amino-2-oxoethylester.

9. Verfahren zur Herstellung einer Verbindung der Formel (I) nach einem der Ansprüche 1 bis 8, bei dem man eine Verbindung der Formel (IV) 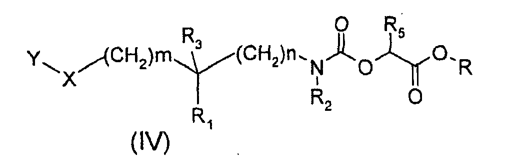 worin Y, X, R₁, R₂, R₃, R₅, m und n die in einem der Ansprüche 1 bis 8 angegebene Bedeutung besitzen und R für eine Methyl- oder Ethylgruppe steht, durch Aminolyse mit einem Amin der Formel R₆NH₂, worin R₆ die in Formel (I) nach Anspruch 1 angegebene Bedeutung besitzt, umwandelt.

10. Pharmazeutische Zusammensetzung, enthaltend mindestens eine Verbindung der Formel (I) nach einem der Ansprüche 1 bis 8 in pharmazeutisch unbedenklicher Basen-, Salz-, Hydrat- oder Solvatform und gegebenenfalls einen oder mehrere pharmazeutisch unbedenkliche Trägerstoffe.

11. Verbindung der Formel (I) nach einem der Ansprüche 1 bis 8 in pharmazeutisch unbedenklicher Basen-, Salz-, Hydrat- oder Solvatform zur Verwendung als Arzneimittel.

12. Verwendung einer Verbindung der Formel (I) nach einem der Ansprüche 1 bis 8 in pharmazeutisch unbedenklicher Basen-, Salz-, Hydrat- oder Solvatform zur Herstellung eines Arzneimittels zur Prävention oder Behandlung einer Pathologie, an der endogene Cannabinoide und/oder andere durch das Enzym FAAH metabolisierte Substanzen beteiligt sind.

13. Verwendung einer Verbindung der Formel (I) nach einem der Ansprüche 1 bis 8 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.
